(19) Europäisches Patentamt
European Patent Office
Office européen des brevets

(11) Veröffentlichungsnummer: **0 044 992**
**A1**

(12) # EUROPÄISCHE PATENTANMELDUNG


(21) Anmeldenummer: **81105441.0**

(22) Anmeldetag: **13.07.81**

(51) Int. Cl.³: **C 07 D 501/20,** C 07 D 499/78, A 61 K 31/545, A 61 K 31/43 // C07D498/04, C07D239/48

(30) Priorität: **26.07.80 DE 3028451**

(43) Veröffentlichungstag der Anmeldung: **03.02.82 Patentblatt 82/5**

(84) Benannte Vertragsstaaten: **AT BE CH DE FR IT LI LU NL SE**



(71) Anmelder: **Dr. Karl Thomae GmbH, Postfach 1755, D-7950 Biberach (Riss) (DE)**

(72) Erfinder: **Wetzel, Bernd, Dr., Dipl.-Chem., Kapellenweg 21, D-7950 Biberach 1 (DE)**
Erfinder: **Woitun, Eberhard, Dr., Dipl.-Chem., Stecherweg 17, D-7950 Biberach 1 (DE)**
Erfinder: **Reuter, Wolfgang, Dr., Dipl.-Chem., Nelkenweg 10, D-7951 Laupertshausen (DE)**
Erfinder: **Maier, Roland, Dr., Dipl.-Chem., Bodelschwinghstrasse 39, D-7950 Biberach 1 (DE)**
Erfinder: **Lechner, Uwe, Dr., Panoramastrasse 12, D-7951 Ummendorf (DE)**
Erfinder: **Goeth, Hanns, Dr., Oberer Bühl 6, D-7950 Biberach 1 (DE)**


(54) **Neue Lactame, Verfahren zu ihrer Herstellung und diese Verbindungen enthaltende Arzneimittel.**


(57) Beschrieben werden neue $\beta$-Lactame der allgemeinen Formel

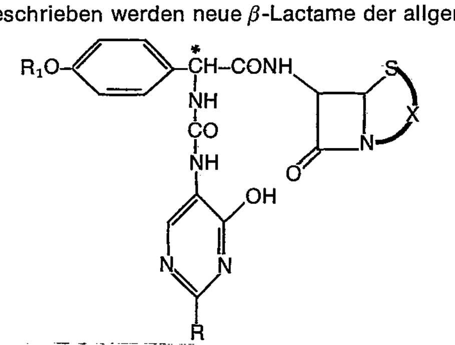

(I)

worin $R_1$ eine aliphatische Acyl- oder Alkoxycarbonylgruppe oder die Aminocarbonylgruppe und X die Gruppen

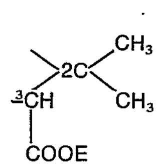

und

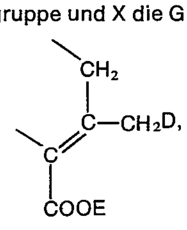

darstellen, wobei D ein Wasserstoffatom, eine Acetoxy-, Aminocarbonyloxy-, Pyridinum- oder 4-Aminocarbonylpyridiniumgruppe oder eine Gruppe –SHet (Het ist beispielsweise der 1-Methyl-tetrazol-5-ylrest) darstellt und E ein Wasserstoffatom oder eine in vitro oder in vivo leicht abspaltbare Schutzgruppe bedeutet, und R ein Wasserstoffatom, die Cyclopropylgruppe, eine niedrige Hydroxyalkylaminogruppe, eine Amino-, Alkyl- oder Alkenylaminogruppe, eine Cycloalkylaminogruppe oder eine Gruppe der allgemeinen Formel

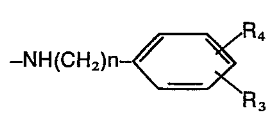

bedeutet, wobei n die Zahlen 0 oder 1 und $R_3$ und $R_4$ Wasserstoffatome, Hydroxy-, Acetylamino-, Aminocarbonylamino-, Nitro-, Aminocarbonyl-, Cyan-, Methylsulfinyl-, Methylsulfonyl-, Aminosulfonyl- oder Methylaminosulfonylgruppen darstellen.


(Fortsetzung nächste Seite)

EP 0 044 992 A1

Es werden des weiteren ihre Salze mit anorganischen oder organischen Basen, 3 Verfahren zur Herstellung dieser Verbindungen und diese Verbindungen enthaltende Arzneimittel beschrieben.

Die Verbindungen der allgemeinen Formel I wirken sowohl gegen grampositive als auch besonders gegen gramnegative Bakterien und gegen bakterienähnliche Mikroorganismen; die Verbindungen zeichnen sich durch eine starke Wirkung und ein breites Wirkungsspektrum aus.

0044992

Case 5/797
Dr. Bu/pf/Kp

DR. KARL THOMAE GMBH, BIBERACH AN DER RISS

## Neue Lactame, Verfahren zu ihrer Herstellung und diese Verbindungen enthaltende Arzneimittel

Die Erfindung betrifft neue ß-Lactame der allgemeinen Formel I

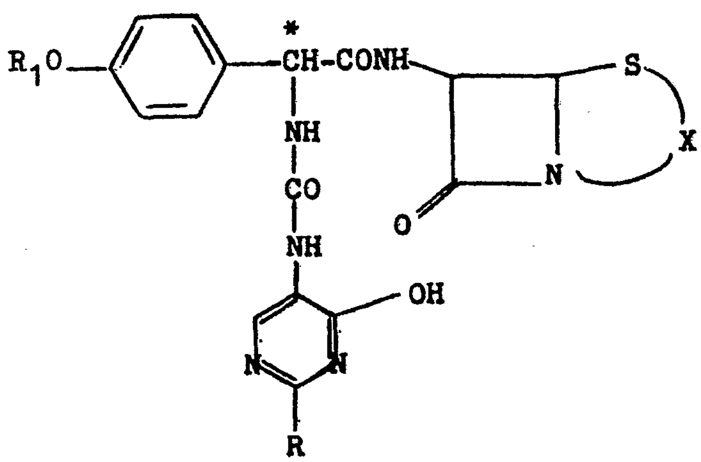

,(I)

gegebenenfalls ihre physiologisch verträglichen Salze mit anorganischen oder organischen Basen, Verfahren zur Herstellung der Verbindungen und diese Verbindungen enthaltende Arzneimittel.

In der allgemeinen Formel I bedeuten:

$R_1$ eine aliphatische Acyl- oder Alkoxycarbonylgruppe mit 2 bis 5 Kohlenstoffatomen oder die Aminocarbonylgruppe,

X die Gruppen

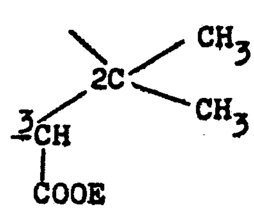 und 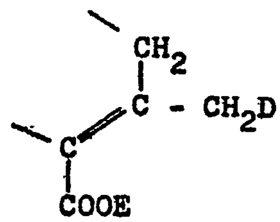 ,

worin

D ein Wasserstoffatom, eine Acetoxy-, Aminocarbonyloxy-, Pyridinium oder 4-Aminocarbonyl-pyridiniumgruppe oder die Gruppe -SHet bedeutet, wobei Het die 1-Methyl-tetrazol-5-yl-, 1,2,4-Thiadiazol-5-yl-, 3-Methyl-1,2,4-thiadiazol-5-yl-, 1,3,4-Thiadiazol-2-yl-, 2-Methyl-1,3,4-thiadiazol-5-yl- oder die 4-Methyl-5,6-dioxo-1,2,4-triazin-3-yl-gruppe darstellt und

E ein Wasserstoffatom oder eine in vitro oder in vivo leicht spaltbare Schutzgruppe;

R ein Wasserstoffatom, die Cyclopropylgruppe, die 2'-Hydroxyäthylamino-, die 3'-Hydroxypropylamino- oder die 4'-Hydroxycyclohexylaminogruppe oder eine Gruppe der allgemeinen Formel

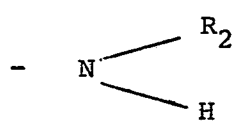 , worin $R_2$ Wasserstoff, eine verzweigte oder unverzweigte Alkyl- oder Alkenylgruppe mit 1 bis 4 Kohlenstoffatomen oder einen Cycloalkylrest mit 3 bis 6 Kohlenstoffatomen bedeutet,

R bedeutet weiter eine Gruppe der allgemeinen Formel

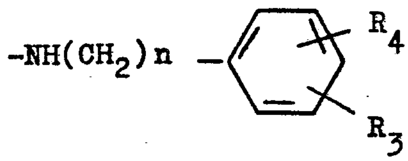

worin n = o oder 1 und $R_3$ und $R_4$, die gleich oder verschieden sein können, Wasserstoffatome, die Hydroxy-, Acetylamino-, Aminocarbonylamino-, Nitro-, Aminocarbonyl-, Cyan-, Methylsulfinyl-, Methylsulfonyl-, Aminosulfonyl- oder die Methylaminosulfonylgruppe bedeuten.

Als Carboxylschutzgruppen E kommen im Sinne der Erfindung solche in Frage, welche auch bisher schon auf dem Gebiet der Penicilline und Cephalosporine eingesetzt wurden, insbesondere esterbildende Gruppen, die durch Hydrogenolyse oder Hydrolyse oder andere Behandlungen unter milden Bedingungen entfernt werden können oder esterbildende Gruppen, welche leicht im lebenden Organismus abgespalten werden können. Beispiele für in vitro leicht spaltbare Schutzgruppen sind z.B. die Benzyl-, Diphenylmethyl-, Trityl-, t-Butyl-, die 2,2,2-Trichloräthyl- oder die Trimethylsilylgruppe.

Bedeutet E Wasserstoff, so fallen unter den Anspruch auch pharmazeutisch verträgliche Salze, wie z.B. Alkali- oder Erdalkalisalze, z.B. Natrium-, Kalium-, Magnesium- oder Calciumsalze, Ammoniumsalze, organische Aminsalze, z.B. mit Triäthylamin oder Dicyclohexylamin.

Steht D für Pyridinium oder Aminocarbonylpyridinium, dann haben die Verbindungen der Erfindung die allgemeine Formel Ia

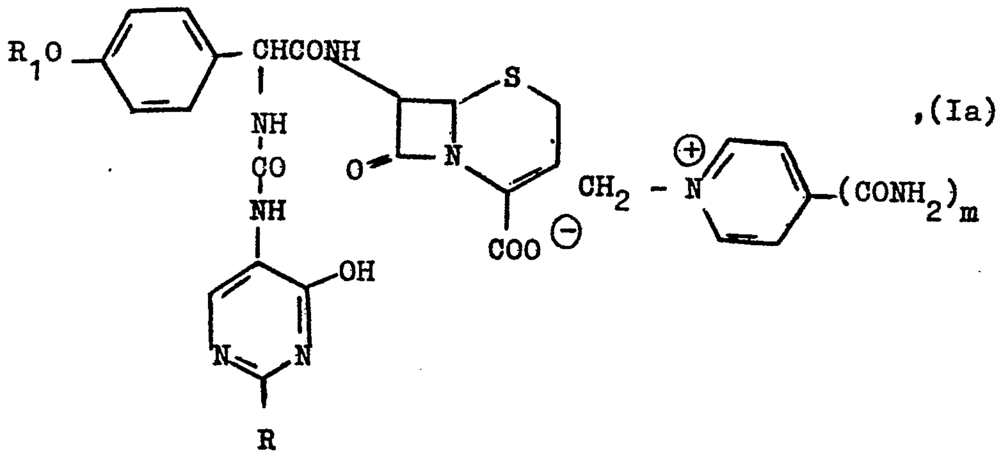

,(Ia)

in der m die Zahl 0 oder 1 bedeutet.

Bevorzugt sind diejenigen Verbindungen der allgemeinen Formel I, worin

$R_1$ eine Methoxycarbonyl-, Äthoxycarbonyl-, Acetyl- oder Aminocarbonylgruppe bedeutet,

X wie oben definiert ist und in X
E ein Wasserstoffatom und
D ein Wasserstoffatom, die Acetoxygruppe oder die Gruppe SHet darstellen, wobei Het die 1-Methyltetrazol-5-yl-, die 1,3,4-Thiadiazol-5-yl- oder die 2-Methyl-1,3,4-thiadiazol-5-yl-gruppe bedeutet; und

R die oben genannten Bedeutungen hat.

Eine besonders bevorzugte Ausführungsform der Erfindung besteht darin, daß R für die Gruppe der allgemeinen Formel

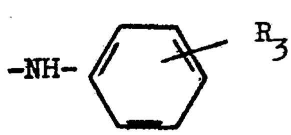

steht,

worin
$R_3$ die Hydroxy-, Methylsulfinyl-, Methylsulfonyl-, Aminocarbonyl-, Aminocarbonylamino-, Aminosulfonyl- oder Methylaminosulfonylgruppe bedeutet,
oder daß R die
m-Hydroxy-p-aminosulfonylanilino-, Cyclopropyl-, Propylamino-, Isopropylamino-, Cyclopentylamino-, Cyclohexylamino-, 3'-Hydroxy-propylamino-, 4'-Hydroxycyclohexylaminogruppe darstellt, und $R_1$ und X wie vorstehend genannt definiert sind.

Die ß-Lactame der allgemeinen Formel I können in zwei tautomeren Formen (nämlich vom Lactim- und von Lactamtyp) vorliegen. Es hängt besonders vom jeweiligen Lösemittel und von der Art des Substituenten R ab, welche der beiden Formen I oder I' überwiegt:

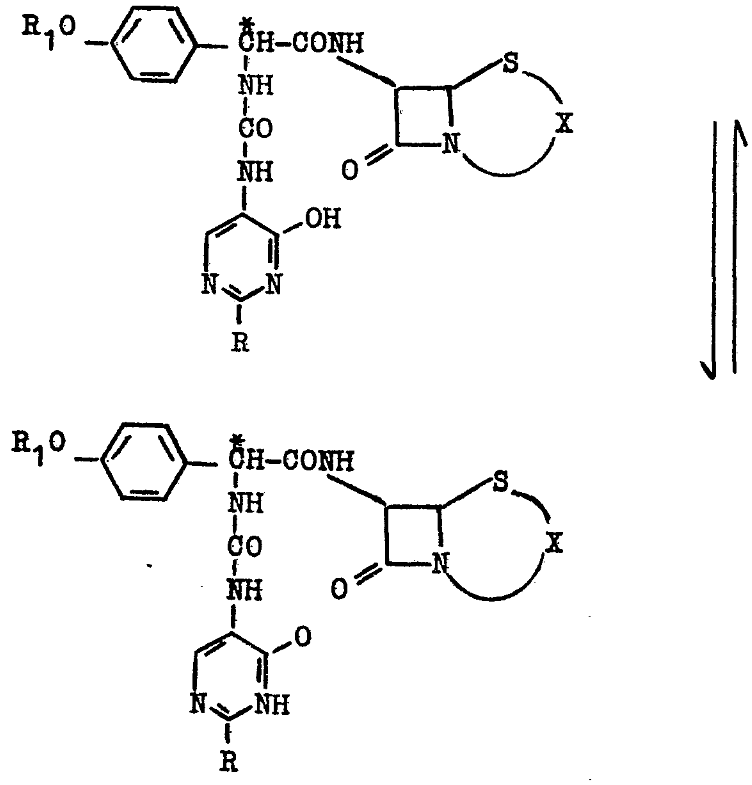

,(I)

,(I')

Es versteht sich von selbst, daß die eingangs angegebenen Verbindungen der allgemeinen Formel I immer beide Tautomeren umfassen.

Die Verbindungen der allgemeinen Formel I können bezüglich des Chiralitätszentrums C+ in den beiden möglichen R- und S-Konfigurationen, jedoch auch als ein Gemisch dieser beiden Konfigurationen, vorliegen. Besonders bevorzugt sind solche Verbindungen, für welche die D=R-Konfiguration zutrifft. Wenn das Endprodukt in der D,L-Form anfällt, gelingt es, die reinen D- und L-Diastereomeren durch präparative Flüssigkeitschromatographie (HPCL) herzustellen.

Die Verbindungen der allgemeinen Formel I können wie folgt hergestellt werden:

1. Verbindungen der allgemeinen Formel I, in der D die angegebene Bedeutung mit Ausnahme der einer Pyridinium- oder Aminocarbonylpyridiniumgruppe besitzt, durch Umsetzung einer Verbindung der allgemeinen Formel

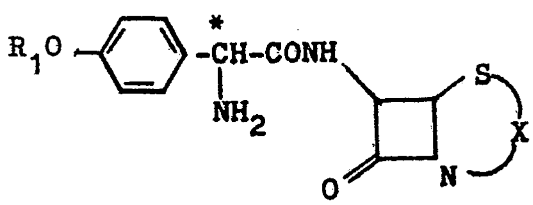

,(II)

in der $R_1$ und X die oben angegebene Bedeutung und D die oben angegebene Bedeutung mit Ausnahme der einer Pyridinium- oder Aminocarbonylpyridiniumgruppe besitzen, mit einem Pyrimidinderivat der allgemeinen Formel

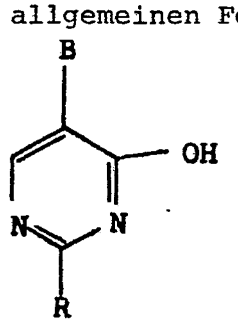

, (III)

in der R wie oben definiert ist und B die Gruppe -NCO oder ein reaktives Derivat der Gruppe -NHCOOH bedeutet, wie z.B.

die Gruppen -NHCOCl, -NHCOBr oder -NH-COO-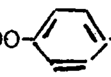-$NO_2$,

wobei die Gruppen -NCO und -NHCOCl besonders bevorzugt sind. Es können auch Gemische von solchen Pyrimidinderivaten der allgemeinen Formel III verwendet werden, in der B teils die eine und teils die andere der vorstehend genannten Bedeutungen besitzt, z.B. die Gruppen -NCO und $-\underset{H}{\underset{|}{N}}-COCl$ gleichzeitig nebeneinander.

Bedeutet E ein Wasserstoffatom, so können die Ausgangsprodukte der allgemeinen Formel II in Form ihrer anorganischen oder organischen Salze, z.B. als Triäthylammoniumsalz oder Natriumsalz, eingesetzt werden. Die Reaktion kann dann in beliebigen Mischungen von Wasser mit solchen organischen Lösungsmitteln, die mit Wasser mischbar sind, wie Ketonen, z.B. Aceton, cyclische Äther, z.B. Tetrahydrofuran oder Dioxan, Nitrilen, z.B. Acetonitril, Formamiden, z.B. Dimethylformamid, Dimethylsulfoxid oder Alkoholen z.B. Isopropanol oder in Hexametapol durchgeführt werden. Dabei hält man den pH der Reaktionsmischung durch Zusatz von Basen oder Verwendung von Pufferlösungen in einem pH-Bereich von etwa 2,0 bis 9,0, vorzugsweise zwischen pH 6,5 und 8,0. Es ist aber auch möglich, die Reaktion in wasserfreien organischen Lösungsmitteln, z.B. halogenierten Kohlenwasserstoffen, wie Chloroform oder Methylenchlorid unter Zusatz von Basen, vorzugsweise Triäthylamin, Diäthylamin oder N-Äthylpiperidin durchzuführen. Weiterhin läßt sich die Reaktion in einem Gemenge aus Wasser und einem mit Wasser nicht mischbaren Lösungsmittel, wie Äther, z.B. Diäthyläther, halogenierten Kohlenwasserstoffen, z.B. Chloroform oder Methylenchlorid, Schwefelkohlenstoff, Ketonen, z.B. Isobutylmethylketon, Estern, z.B. Essigsäureäthylester, aromatischen Lösungsmitteln, z.B. Benzol ausführen, wobei es zweckmäßig ist, kräftig zu rühren, und den pH-Wert durch

Basenzusatz oder Verwendung von Pufferlösungen in einem Bereich von etwa pH 2,0 bis 9,0, vorzugsweise zwischen 6,5 und 8,0, zu halten. Man kann die Reaktion aber auch in Wasser allein in Gegenwart einer organischen oder anorganischen Base oder unter Zusatz von Pufferstoffen durchführen.

Bedeutet E die Trimethylsilylgruppe, d.H. verwendet man als Ausgangsprodukte für das erfindungsgemäße Verfahren die Silylderivate der Verbindungen der allgemeinen Formel II (z.B. Mono- oder zweckmäßigerweise die an der Amino- und Carboxylgruppe silylierten Di-trimethylsilylderivate) und setzt sie mit Verbindungen der allgemeinen Formel III um, so arbeitet man im allgemeinen zweckmäßigerweise in wasser- und hydroxyl-gruppenfreien Lösungsmitteln, beispielsweise in halogenierten Kohlenwasserstoffen, z.B. Methylenchlorid oder Chloroform, Benzol, Tetrahydrofuran, Aceton oder Dimethylformamid usw. Der Zusatz von Basen ist hierbei nicht notwendig, kann jedoch in einzelnen Fällen vorteilhaft sein, um die Ausbeute und Reinheit der Produkte zu verbessern. Als gegebenenfalls zugesetzte Basen werden zweckmäßigerweise tertiäre aliphatische oder aromatische Amine, wie Pyridin oder Triäthylamin, oder durch sterische Hinderung schwer acylierbare sekundäre Amin, wie Dicyclohexylamin, benützt. Bedeutet E eine der anderen obengenannten in vitro oder in vivo leicht spaltbaren Schutzgruppen, z.B. die Diphenylmethylestergruppe oder die Pivaloyloxymethylgruppe, so empfiehlt es sich ebenfalls in einem aprotischen Lösungsmittel zu arbeiten, z.B. in absolutem Methylenchlorid, Chloroform, Tetrahydrofuran oder Dimethylformamid.

Die Menge der verwendeten Basen ist z.B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich oder nicht sinnvoll ist, werden im Falle der Verwendung der nichtsilylierten Verbindungen der allgemeinen Formel II vorzugsweise 1,0 bis 2,0 Moläquivalente Basen eingesetzt. Im Falle der Verwendung der silylierten Verbindungen verwendet man vorzugsweise bis zu einem Moläquivalent Base.

Als Basen können prinzipiell alle in der organischen Chemie üblicherweise verwendeten organischen und anorganischen Basen verwendet werden, wie Alkali- und Erdalkalihydroxide, Erdalkalioxide, Alkali- und Erdalkalicarbonate und Hydrogencarbonate, Ammoniak, primäre, sekundäre und tertiäre aliphatische und aromatische Amine sowie heterocyclische Basen. Beispielhaft seien Natrium-, Kalium- und Calziumhydroxid, Calziumoxid, Natrium- und Kaliumcarbonat, Natrium- und Kaliumhydrogencarbonat, Diäthylamin, Methyl-äthylamin, Triäthylamin, Hydroxyäthylamin, Anilin, Dimethylanilin, Pyridin und Piperidin genannt. Bei Verwendung der silylierten Ausgangsstoffe sollten jedoch die oben angegebenen Einschränkungen bezüglich der Art der Basen beachtet werden.

Als Puffersysteme können alle üblichen Puffermischungen verwendet werden, z.B. Phosphatpuffer, Citratpuffer und Tris-(hydroxymethyl)-amino-methan-Puffer.

Die Reaktionstemperaturen können in einem größeren Bereich variiert werden. Im allgemeinen arbeitet man zwischen -20 und +50°C, vorzugsweise zwischen 0 und +20°C.

Die Reaktionspartner der allgemeinen Formeln II und III können von vornherein in äquimolaren Mengen miteinander zur Reaktion gebracht werden. Es kann aber in einzelnen Fällen durchaus zweckmäßig sein, einen der beiden Reaktionspartner im Überschuß zu verwenden, um sich damit die Reinigung des Endproduktes zu erleichtern oder um die Ausbeute zu steigern.

2. Verbindungen der allgemeinen Formel I, in der D die oben angegebenen Bedeutungen mit Ausnahme der einer Pyridinium- oder Aminocarbonylpyridiniumgruppe besitzt, durch Umsetzung von Ureidocarbonsäuren der allgemeinen Formel IV,

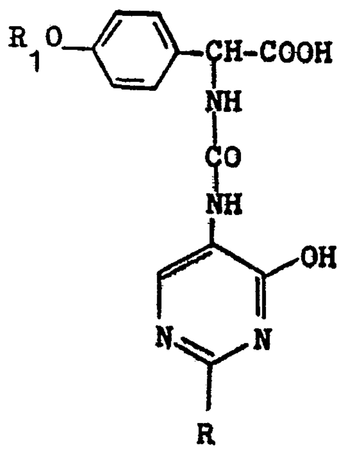

,(IV)

in der R und $R_1$ die oben angegebenen Bedeutungen besitzen, ihren Salzen oder reaktiven Derivaten, mit Verbindungen der allgemeinen Formel V,

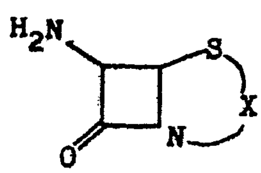

,(V)

in der X die oben angegebenen Bedeutungen, mit Ausnahme der Formel, in der D für Pyridinium oder Aminocarbonylpyridinium steht, hat.

Als reaktive Derivate der Ureidocarbonsäuren der allgemeinen Formel IV kommen beispielsweise deren Säureanhydride wie z.B. die, die sich von Chlorameisensäureestern, z.B. Chlorameisensäureäthyl- oder isobutylester, ableiten, oder deren reaktive Ester, wie der p-Nitrophenylester oder der N-Hydroxysuccinimidester, oder deren reaktive Amide, wie das N-Carbonylimidazol, aber auch deren Säurehalogenide, wie das entsprechende Säurechlorid oder deren Säureazide in Frage.

Prinzipiell können jedoch alle Verknüpfungsmethoden, wie sie aus der ß-Lactamchemie bekannt sind, verwendet werden.

Die 7-Aminocephalosporansäure- oder Penicillansäurederivate der allgemeinen Formel V werden vorteilhafterweise in Form eines in vitro oder in vivo leicht spaltbaren Derivates eingesetzt. Hierfür kommen z.B. die Verbindungen der allgemeinen Formel V in Frage, bei denen E die eingangs gegebenen Bedeutungen mit Ausnahme der eines Wasserstoffatoms besitzt; besonders bevorzugte Derivate sind der Diphenylmethylester, der t-Butylester, der Trimethylsilylester oder das N,O-Bistrimethylsilylderivat.

Beispielsweise werden die Ureidocarbonsäuren, ihre Salze oder ihre reaktiven Derivate mit den 7-Aminocephalosporan- oder 6-Aminopenicillansäurederivaten in einem Lösungsmittel bei Temperaturen zwischen -40°C und +40°C, gegebenenfalls in Gegenwart einer Base, umgesetzt. Wird z.B. ein Anhydrid der Ureidocarbonsäure, beispielsweise das Anhydrid mit dem Äthylchloroformiat, eingesetzt, so wird die Reaktion unter Kühlung beispielsweise bei -40°C bis +10°C in einem Lösungsmittel, wie Aceton, Tetrahydrofuran, Dimethylformamid, Chloroform, Dichlormethan, Hexametapol oder in einem Gemisch dieser Lösungsmittel, durchgeführt. Setzt man beispielsweise einen N-Hydroxy-succinimidester der Ureidocarbonsäure mit Derivaten der allgemeinen Formel V um, so wird die Reaktion vorzugsweise bei 0 bis 20°C in Anwesenheit einer Base, wie z.B. Triäthylamin, in einem Lösungsmittel wie Dimethylformamid, Dichlormethan, Dioxan oder in einem Gemisch solcher Lösungsmittel durchgeführt.

Die Umsetzung einer Ureidocarbonsäure der allgemeinen Formel IV selbst oder ihrer Salze mit Verbindungen der allgemeinen Formel V erfolgt vorteilhafterweise in Gegenwart eines Kondensationsmittels, z.B. in Gegenwart von N,N'-Dicyclohexylcarbodiimid.

3. Cephalosporinderivate der allgemeinen Formel I, in der D die Bedeutungen einer -S-Het-, Pyridinium- oder 4-Aminocarbonylpyridiniumgruppe besitzt, durch Umsetzung einer Verbindung der allgemeinen Formel VI,

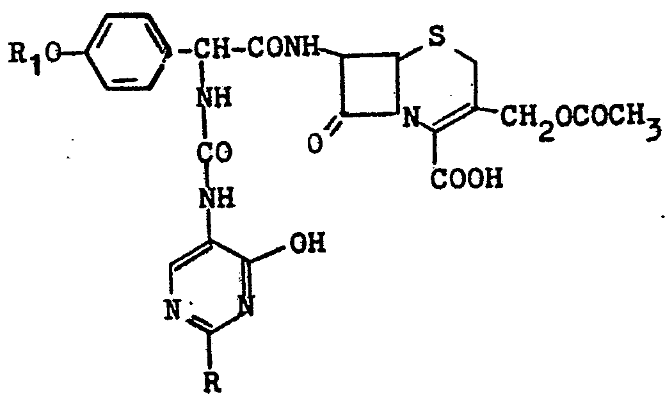

,(VI)

in der
$R_1$ und R die oben angegebenen Bedeutungen haben, entweder mit einer Verbindung der allgemeinen Formel VII,

Het - S - M ,(VII)

in der
Het die oben angegebenen Bedeutungen hat und M für ein Wasserstoffatom oder ein Alkalimetall oder ein Erdalkalimetall steht oder mit Pyridin bzw. 4-Aminocarbonylpyridin. Dazu wird z.B. eine Verbindung der Formel VI mit beispielsweise 5-Methyl-2-mercapto-1,3,4-thiadiazol in einem Lösungsmittel,

wie z.B. Wasser, Methanol, Äthanol, Aceton, Methyläthylketon, Tetrahydrofuran, Acetonitril, Essigsäureäthylester, Dimethoxyäthan, Dimethylformamid, Dimethylsulfoxid, Chloroform oder einem Gemisch dieser Lösungsmittel umgesetzt. Vorzugsweise wird ein stark polares Lösungsmittel, wie Wasser, Acetonitril oder dergleichen, verwendet: Im Falle von Wasser als Lösungsmittel wird der pH-Wert der Reaktionslösung vorteilhafterweise auf 2 bis 10 und insbesondere auf 4 bis 8 gehalten. Der gewünschte pH-Wert kann durch Zugabe einer Pufferlösung, wie Natriumphosphat, eingestellt werden. Die Reaktionsbedingungen unterliegen keinen besonderen Beschränkungen. Normalerweise wird die Umsetzung bei einer Temperatur im Bereich von $0^{o}$ bis $100^{o}C$ während einer Zeitdauer von einigen Stunden durchgeführt.

Die nach den Verfahren 1 - 3 hergestellten erfindungsgemäßen Verbindungen, in denen E für eine in vitro leicht abspaltbare Schutzgruppe steht, können nach bekannten Methoden der Cephalosporin- oder Penicillinchemie in die freien Carbonsäuren (E = Wasserstoff) der allgemeinen Formel I überführt werden. So kann beispielsweise die Trimethylsilylgruppe leicht durch wäßrige Hydrolyse entfernt werden oder die Diphenylmethylestergruppe z.B. durch hydrolytische Spaltung mit Trifluoressigsäure. Diese Eliminierung der Schutzgruppen ist allgemein bekannt.

Ebenso können die Antibiotika der Formel I bzw. I', worin E ein Wasserstoffatom bedeutet, in Acyloxyalkylester, worin E z.B. einen Pivaloyloxymethylrest

$$-CH_2-\underset{\underset{O}{\|}}{OC}-C(CH_3)_3$$

bedeutet, überführt werden, indem man ein Alkalisalz der freien Carbonsäure, beispielsweise ein Natrium- oder Kaliumsalz, mit einem Pivaloyloxymethylhalogenid der Formel

$$Hal\text{-}CH_2\text{-}O\text{-}\underset{\underset{O}{\|}}{C}\text{-}C(CH_3)_3$$

worin Hal für Chlor, Brom oder Jod steht, umsetzt. Weitere geeignete Acyloxyalkylhalogenide sind z.B. Chlormethylacetat, Brommethylpropionat oder 1-Bromäthylacetat.

Die Herstellung der Acyloxyalkylester der Formel I wird vorgenommen, indem man das jeweilige Alkalisalz der Stammsäure in einem inerten Lösungsmittel mit einem leichten molaren Überschuß des Jod-, Brom- oder Chloralkylesters, wie Pivaloyloxymethyljodid, bei Raumtemperatur oder leicht erhöhter Temperatur bis zu etwa 40 bis 45°C umsetzt. Als Lösungsmittel können beispielsweise Aceton, Tetrahydrofuran, Dioxan, Dimethylformamid oder Methylenchlorid verwendet werden.

Die Aufarbeitung der nach den genannten Verfahren erhaltenen Reaktionsgemische nach erfolgter Umsetzung wird nach den bei ß-Lactam-Antibiotica gebräuchlichen Methoden vorgenommen; dasselbe gilt für die Isolierung und Reinigung der Endprodukte, beispielsweise für die Freisetzung der Säure oder ihre Überführung in andere Salze mittels anorganischer oder organischer Basen. Für die Herstellung der Kalium- oder Natriumsalze bewährt sich besonders die Fällung dieser Salze aus einer alkoholisch-ätherischen Lösung der freien Säure durch die Zugabe von Kalium- oder Natrium-2-äthylhexanoat oder die Umsetzung der freien Säure mit der entsprechenden Menge Natriumbicarbonat unter pH-Kontrolle und Kühlung sowie anschließender Gefriertrocknung.

Die Ausgangsstoffe der Formel II sind bekannt oder können in Analogie zu bekannten Stoffen nach an sich bekannten Methoden hergestellt werden, z.B. durch Acylierung der bekannten Aminolactame der Formel IV und, falls erwünscht, anschließende Umsetzung so erhaltener Cephalosporansäurederivate der Formel II (D= $-OCOCH_3$) mit Thiolen der Formel Het-SH.

Die Ausgangsstoffe der allgemeinen Formel III können beispielsweise durch Umsetzung der entsprechenden 5-Aminopyrimidine der allgemeinen Formel VIII,

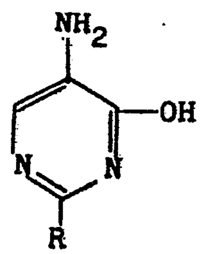

,(VIII)

in der
R wie oben definiert ist, mit Phosgen gewonnen werden. Diese Umsetzung erfolgt vorzugsweise in einem nicht Hydroxylgruppen enthaltenden Lösungsmittel, wie Tetrahydrofuran, Methylenchlorid, Chloroform, Dimethoxyäthan oder Hexametapol bei Temperaturen zwischen -40$^{o}$ und +60$^{o}$C, vorzugsweise zwischen -10$^{o}$ und +20$^{o}$C. Dabei empfiehlt es sich, den entstehenden Chlorwasserstoff durch äquimolare Mengen einer inerten organischen Base, wie Triäthylamin oder Pyridin, zu binden. Als Lösungsmittel kann auch Pyridin im Überschuß verwendet werden. Sollten die betreffenden Aminopyrimidine der allgemeinen Formel VIII in einem der erwähnten Lösungsmittel schwer löslich sein, kann die Phosgenierung auch in heterogener Phase durchgeführt werden. Desweiteren können die Aminopyrimidine der allgemeinen Formel VIII durch Behandlung mit einem Silylierungsmittel, wie Hexamethyldisilazan oder Trimethylchlorsilan/Triäthylamin, Trimethylsilyldiäthylamin oder N,O-Bistrimethylsilylacetamid in ein im allgemeinen in den erwähnten Lösungsmitteln sehr leicht lösliches, einfach oder, entsprechend den vorhandenen austauschbaren Wasserstoffatomen,

mehrfach silyliertes Aminopyrimidin überführt werden, das mit Phosgen dann zu den entsprechenden Verbindungen der allgemeinen Formel III reagiert. Je nach der Art des Lösungsmittels, der Höhe der Temperatur, der Menge und Art der eingesetzten Base entsteht entweder überwiegend das entsprechende Isocyanat oder Carbaminsäurehalogenid oder ein Gemisch dieser beiden Verbindungen. Je nach den Reaktionsbedingungen kann die Verbindung der allgemeinen Formel III auch teilweise oder ganz als ein, den Isocyanaten isomeres Dihydrooxazolo-pyrimidin der allgemeinen Formel IIIa,

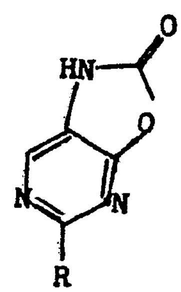

,(IIIa)

oder bei vorhergehender Silylierung je nach Art des Substituenten R als ein- oder mehrfach silyliertes Analoges vorliegen.

Die durch Phosgenierung entstandenen Ausgangsprodukte der allgemeinen Formel III bzw. IIIa oder deren Gemische sind in den obenerwähnten Lösungsmitteln im allgemeinen gut löslich und können, nach Entfernung des überschüssigen Phosgens, ohne weitere Reinigung mit den entsprechenden ß-Lactam-derivaten der allgemeinen Formel II direkt umgesetzt werden.

Es ist jedoch auch möglich, das Zwischenprodukt der allgemeinen Formel IIIa zu isolieren, es gegebenenfalls mit einem protischen Lösemittel, beispielsweise Wasser oder Methanol zu entsilylieren, es auf Grund seiner Löslichkeitseigenschaften zu reinigen und in der oben dargelegten Weise umzusetzen.

Die Ureidocarbonsäuren der allgemeinen Formel IV lassen sich leicht durch Umsetzung der Pyrimidinderivate der allgemeinen Formel III mit Glycinderivaten der allgemeinen Formel IX,

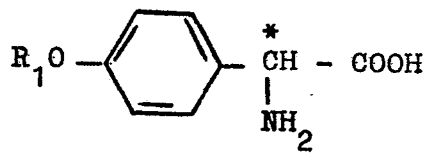
,(IX)

in der

$R_1$ wie oben definiert ist, erhalten. Die Umsetzung erfolgt bei Temperaturen zwischen -20° und +40°C, vorzugsweise zwischen 0° und +20°C, in einem Lösungsmittel. Als Lösungsmittel können hierbei z.B. Gemische von Wasser und organischen Lösungsmitteln, die mit Wasser mischbar sind, verwendet werden, beispielsweise Aceton, Tetrahydrofuran, Dioxan, Acetonitril, Dimethylformamid, Äthanol, Dimethylsulfoxid. Gegebenenfalls wird die Verwendung eines halogenwasserstoffbindenden Mittels notwendig, als solche eignen sich beispielsweise Trialkylamine, wie Triäthylamin oder anorganische Basen, wie verdünnte Natronlauge.

Die 2- substituierten 5-Amino-4-hydroxypyrimidine der allgemeinen Formel VIII werden in der DT-OS 28 08 153.0 und DT-OS 29 10 190.4 beschrieben.

Es wurde gefunden, daß die Verbindungen der allgemeinen Formeln I bzw. I' wertvolle pharmakologische Eigenschaften bei guter Verträglichkeit besitzen. Die erfindungsgemäßen Wirkstoffe können daher zur Prophylaxe und Chemotherapie von lokalen und systemischen Infektionen in der Human- und Tiermedizin verwendet werden. Als Krankheiten, die durch die erfindungsgemäßen Verbindungen verhindert bzw. geheilt werden können, seien beispielsweise solche der Atmungswege, des Rachenraumes und der Harnwege genannt; die Verbindungen wirken insbesondere gegen Pharyngitis, Pneumonie, Peritonitis, Pyelonephritis, Otitis, Cystitis, Endocarditis, Bronchitis, Arthritis und allgemeine systemische Infektionen.

Dieses wird dadurch ermöglicht, daß die Verbindungen der allgemeinen Formeln I bzw. I' sowohl in vitro als auch in vivo gegen schädliche Mikroorganismen, sowohl gegen grampositive als auch besonders gegen gramnegative Bakterien und bakterienähnliche Mikroorganismen sehr stark wirken, wobei sie sich durch ein breites Wirkungsspektrum auszeichnen.

Mit diesen Derivaten können beispielsweise lokale und/oder systemische Erkrankungen behandelt und/oder verhindert werden, die durch die folgenden Erreger oder durch Mischungen der folgenden Erreger verursacht werden:

Micrococcaceae, wie Staphylokokken;
Lactobacteriaceae, wie Streptokokken;
Neisseriaceae, wie Neisserien;
Corynebacteriaceae, wie Corynebakterien;
Enterobacteriaceae, wie Escherichiae-Bakterien der Coli-Gruppe,
Klebsiella-Bakterien, z.B. K. pneumonia;
Proteae-Bakterien der Proteus-Gruppe z.B. Proteus vulgaris;
Salmonella-Bakterien, z.B. S.thyphimurium;
Shigella-Bakterien, z.B. Shigella dysenteriae;
Pseudomonas-Bakterien, z.B. Pseudomonas aeruginosa;
Aeromonas-Bakterien, z.B. Aeromonas lique faciens;
Spirillaceae, wie Vibrio-Bakterien, z.B. Vibrio cholerae;
Parvobateriaceae oder Brucellaceae, wie Pasteurella-Bakterien;
Brucella-Bakterien, z.B. Brucella abortus;
Haemophilus-Bakterien, z.B. Haemophilus influenzae;

Bordetella-Bakterien, z.B. Bordetella pertussis;
Moraxella-Bakterien, z.B. Moraxella lacunata;
Bacteroidaceae, wie Bacteroides-Bakterien;
Fusiforme-Bakterien, z.B. Fusobacterium fusiforme;
Sphaerophorus-Bakterien, z.B. Sphaerophorus necrophorus;
Bacillaceae, wie aerobe Sporenbildner, z.B. Bacillus anthracis;
anaerobe Sporenbildner-Chlostridien, z.B. Chlostridium perfringens;
Spirochaetaceae, wie Borrelia-Bakterien;
Treponema-Bakterien, z.B. Treponema pallidum;
Leptospira-Bakterien, wie Leptospira interrogans.

Die obige Aufzählung von Erregern ist lediglich beispielhaft und keineswegs beschränkend aufzufassen.

In den folgenden Tabellen werden typische, besonders gut wirksame Verbindungen entsprechend der vorliegenden Erfindung aufgezählt. Die Penicilline können nach der Herstellungsmethode 1 und 2, die Cephalosporine nach den Methoden 1 bis 3 gewonnen werden.

Tabelle 1 Penicilline der allgemeinen Formel:

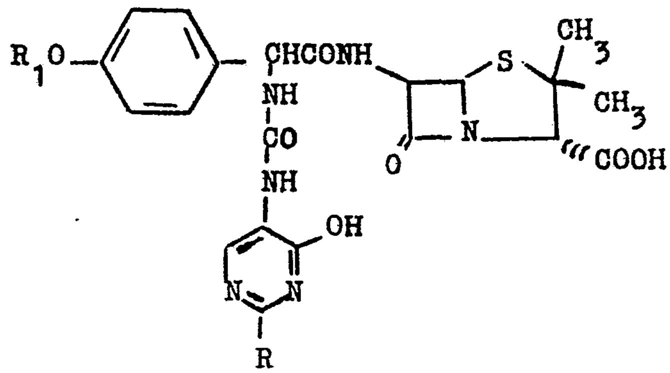

| | $R_1$ | R |
|---|---|---|
| 1) | $CH_3OC-$<br>O | -NH 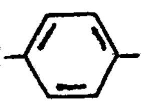 $SO_2NH_2$ |
| 2) | $C_2H_5OC-$<br>O | -NH 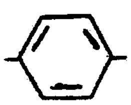 $SO_2NH_2$ |
| 3) | $CH_3C-$<br>O | -NH 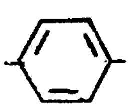 $SO_2NH_2$ |

| | $R_1$ | R |
|---|---|---|
| 4) | $C_3H_7O\underset{O}{\underset{\|}{C}}-$ | 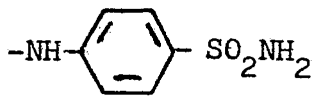 |
| 5) | $(CH_3)_2CH-CH_2O\underset{O}{\underset{\|}{C}}-$ | 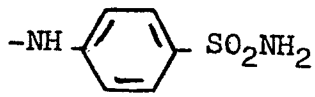 |
| 6) | $C_2H_5-O-\underset{O}{\underset{\|}{C}}-$ | 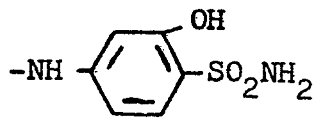 |
| 7) | $CH_3-O-\underset{O}{\underset{\|}{C}}-$ | 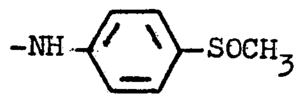 |
| 8) | $C_2H_5-O-\underset{O}{\underset{\|}{C}}-$ | Cyclopropyl |
| 9) | $C_2H_5-O-\underset{O}{\underset{\|}{C}}-$ | 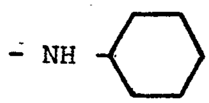 |
| 10) | $C_2H_5-O-\underset{O}{\underset{\|}{C}}-$ | $-NH(CH_2)_3OH$ |
| 11) | $CH_3CO-$ | 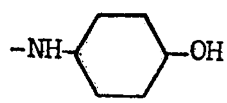 |
| 12) | $CH_3O\underset{O}{\underset{\|}{C}}-$ | 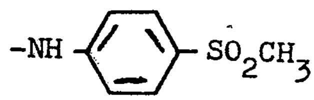 |
| 13) | $H_2N\underset{O}{\underset{\|}{C}}-$ | 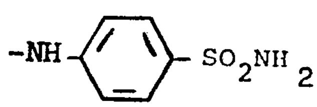 |

Tabelle 2: Cephalosporine der allgemeinen Formel:

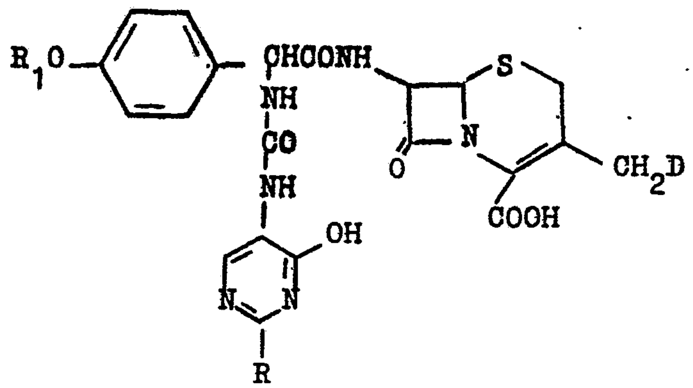

| | $R_1$ | R | D |
|---|---|---|---|
| 1) | $CH_3OC(=O)-$ | 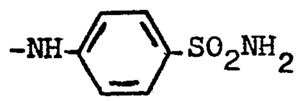 | $-OCO\ CH_3$ |
| 2) | $CH_3OC(=O)-$ | 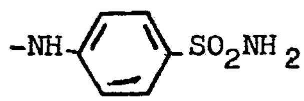 | 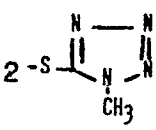 |
| 3) | $C_2H_5OC(=O)-$ | 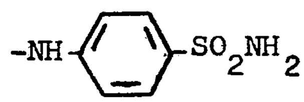 | 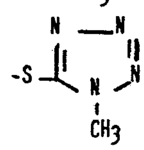 |
| 4) | $C_2H_5-O-C(=O)-$ | 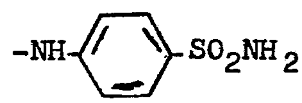 | 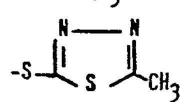 |
| 5) | $C_2H_5-O-C(=O)-$ | 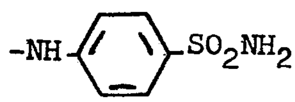 | $-OCO\ CH_3$ |
| 6) | $CH_3CO-$ | 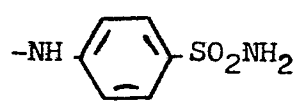 | $-OCO\ CH_3$ |
| 7) | $CH_3CO-$ | 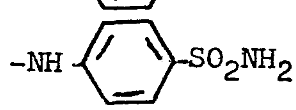 | 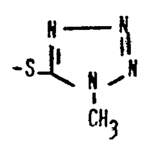 |
| 8) | $C_3H_7OC(=O)-$ | 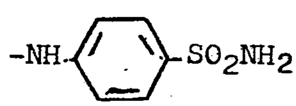 | 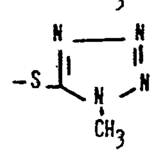 |

| | $R_1$ | R | D |
|---|---|---|---|
| 9) | $C_2H_5-O-\underset{O}{\underset{\|}{C}}-$ | 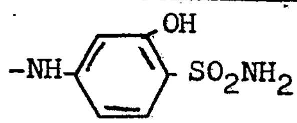 | 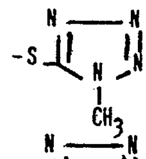 |
| 10) | $CH_3-O-\underset{O}{\underset{\|}{C}}-$ | 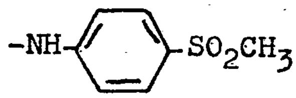 | 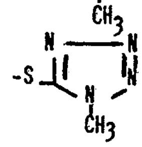 |
| 11) | $CH_3-O-\underset{O}{\underset{\|}{C}}-$ | 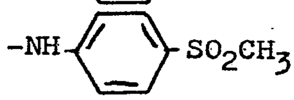 | $-OCOCH_3$ |
| 12) | $C_2H_5-O-\underset{O}{\underset{\|}{C}}-$ | 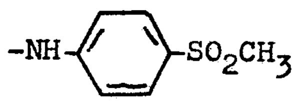 | 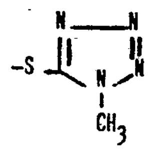 |
| 13) | $C_2H_5-O-\underset{O}{\underset{\|}{C}}-$ | $-NH(CH_2)_3OH$ | $-OCOCH_3$ |
| 14) | $C_2H_5-O-\underset{O}{\underset{\|}{C}}-$ | $-NH(CH_2)_3OH$ | 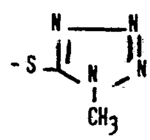 |
| 15) | $C_2H_5-O-\underset{O}{\underset{\|}{C}}-$ | $-NH(CH_2)_3OH$ | 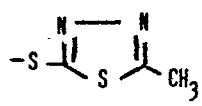 |
| 16) | $CH_3-CO-$ | 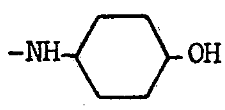 | 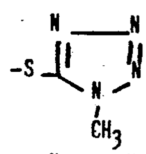 |
| 17) | $CH_3-O-\underset{O}{\underset{\|}{C}}-$ | 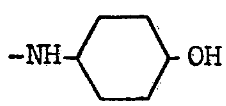 | 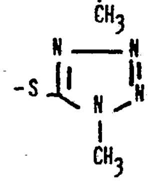 |
| 18) | $C_2H_5-O-\underset{O}{\underset{\|}{C}}-$ | $-NHCH(CH_3)_2$ | 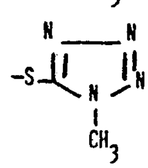 |
| 19) | $H_2N\underset{O}{\underset{\|}{C}}-$ | 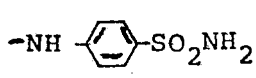 | 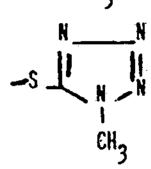 |
| 20) | $H_2N\underset{O}{\underset{\|}{C}}$ | 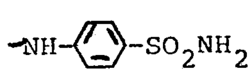 | $-OCOCH_3$ |

Die Wirksamkeit der erfindungsgemäßen ß-Lactam-Antibiotika läßt sich durch folgende Untersuchungen beispielhaft demonstrieren:

1. <u>In vitro-Versuche:</u>

Für die Untersuchungen wurde die Methode des Reihenverdünnungstestes im Mikrotitersystem angewandt. Die Prüfung der Substanzen auf Bakteriostase erfolgte in flüssigem Medium. Es wurde die Bakteriostasewirkung bei folgenden Konzentrationen untersucht:
128, 64, 32, 16, 8, 4, 2, 1, 0,5, 0,25, 0,12, 0,06 µg/ml.
Es wurde ein Nährboden folgender Zusammensetzung benutzt: 10 g Pepton, 8 g Fleischextrakt-Oxoid, 3 g Natriumchlorid, 2 g sek. Natriumphosphat werden mit dest. Wasser auf 100 ml aufgefüllt (pH 7,2 - 7,4). Lediglich bei der Testung gegen Streptokokken wurde 1 % Glucose hinzugefügt. Das Alter der Primärkulturen betrugt etwa 20 Stunden. Die Einstellung der Keimsuspension erfolgte am Photometer (nach "Eppendorf") (Reagenzglas-Durchmesser 14 mm, Filter 546nm) anhand der Trübung einer Bariumsulfat-Vergleichssuspension, die durch eine Bariumsulfat-Aufschwemmung erzeugt wurde, welche durch die Zugabe von 3,0 ml 1%ige Barium-chloridlösung in 97 ml 1%ige Schwefelsäure entstand. Nach der Einstellung wurden Streptococcus Aronson im Verhältnis 1:15 und die übrigen Testkeime im Verhältnis 1:1500 mit einer Kochsalzlösung weiter verdünnt.
16 mg der jeweiligen Substanz wurden in 10 ml-Meßkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension (0,01 ml) beschickt und 18 bis 20 Stunden bei 37°C bebrütet. Eine Lösungsmittelkontrolle wurde stets mitgeführt.

Die Ablesung wurde makroskopisch vorgenommen, wobei die jeweilige Grenzkonzentration (= niedrigste noch bakteriostatisch wirksame Konzentration) ermittelt wurde.

Als Testorganismen wurden benützt:

Staphylococcus aureus SG 511, Escherichia coli ATCC 11 775, Pseudomonas aeruginosa Hamburgensis und Pseudomonas aeruginosa Walter, Serratia marcescens ATCC 13 880, Klebsiella pneumoniae ATCC 10 031 und BC 6, Proteus mirabilis Hamburgensis, Proteus rettgeri, Enterobacter cloacae ATCC 13 047, E. coli R+TEM (ß-Lactamase-Träger).

In der folgenden Tabelle 1 sind die ermittelten minimalen Hemmkonzentrationen (MIC) für typische Vertreter der erfindungsgemäßen Verbindungen aufgeführt:

D-α-[3-(2-p-Aminosulfonylanilino-4-hydroxy-5-pyrimidinyl)-ureido]-p-äthoxycarbonyloxy-benzylpenicillin-Natrium = A

D-α-[3-(2-p-Aminosulfonylanilino-4-hydroxy-5-pyrimidinyl)-ureido]-p-methylcarbonyloxy-benzylpenicillin-Natrium = B

Natrium-7-{D-α-[3-(2-p-aminosulfonylanilino-4-hydroxy-5-pyrimidinyl)-ureido]-p-äthoxy-carbonyloxy-phenylacetamido}-3-[(1-methyl-tetrazol-5-yl)-thiomethyl]-ceph-3-em-4-carboxylat = C

Natrium-7-{D-α-[3-(2-p-aminosulfonyl-anilino-4-hydroxy-5-pyrimidinyl)-ureido]-p-methylcarbonyloxy-phenylacetamido}-3-[(1-methyl-tetrazol-5-yl)-thiomethyl]-ceph-3-em-4-carboxylat = D

Minimale Hemmkonzentration in µg/ml

| Verbindungen | Staph. aureus SG 511 | E.coli ATCC 11775 | Pseud. Hbg. | Pseud.W. | Serr. marcesc. ATCC 13880 | Kl.pneum. ATCC 10031 | Kl.pneum. BC 6 | Prot. mirab. Hbg. | Prot. Rettg. | Ent. cloacae ATCC 13047 | E.coli R+TEM |
|---|---|---|---|---|---|---|---|---|---|---|---|
| A | 0,5 | 0,25 | 2 | 2 | 0,25 | 2 | 2 | 0,12 | 1 | 1 | >64 |
| B | 0,5 | 0,25 | 4 | 2 | 0,5 | 4 | 2 | 0,12 | 2 | 2 | >64 |
| C | 1 | 0,12 | 8 | 4 | 0,25 | 0,5 | 0,25 | 0,12 | 1 | 0,5 | 4 |
| D | 1 | 0,5 | 16 | 8 | 2 | 2 | 1 | 0,25 | 2 | 4 | 16 |
| Azlo-cillin | 2 | 8-16 | 8 | 8 | 4 | 32 | 32 | 2 | 8 | 32 | >64 |
| Cefur-oxim | 1 | 8 | >128 | >128 | 8 | 2 | 4 | 0,5 | 2 | 32 | 4 |

Die genannten Verbindungen sind damit den Vergleichssubstanzen in ihrer Wirksamkeit gegenüber typischen gramnegativen Hospitalkeimen bei Erhalt der Wirkung gegen grampositive Keime deutlich überlegen.

Die akute Toxizität wurde durch perorale und subcutane Applikation der Verbindungen der Tabelle 1 und 2 an der Ratte in steigenden Dosen bestimmt.

Die $LD_{50}$ ist die Dosis, nach deren Applikation 50 % der Tiere innerhalb von 8 Tagen sterben. Sämtliche Substanzen zeigten bei oraler Gabe eine $LD_{50}$ von über 4 g/kg, bei subcutaner Gabe eine $LD_{50}$ von über 3 g/kg, d.h. bei 3 g/kg starben keine Tiere, sie sind damit für die Praxis untoxisch.

Eine Reihe von erfindungsgemäßen Verbindungen wurde in vivo bei experimentellen Infektionen an Mäusen untersucht. Man verwendete als pathogene Bakterien E. coli ATCC 11 775. Es wurde eine intraperitoneale Infektion mit 0,2 ml einer Bakteriensuspension (mit 5 % Mucin) gesetzt. Dies entspricht etwa 1,4 x $10^6$ Keime E. coli/Maus. Weibliche Mäuse vom Stamm NMRI wurden in Gruppen von jeweils 10 Tieren aufgeteilt, zwei Gruppen blieben unbehandelt, die restlichen Gruppen wurden mit verschiedenen Dosen der jeweiligen erfindungsgemäßen Penicilline und Cephalosporine subcutan zur Bestimmung der $ED_{50}$ (Dosis bei der 50 % der Tiere überleben) behandelt. Es wurde einmal eine Stunde nach Setzen der Injektion therapiert.

Der Beobachtungszeitraum betrug in beiden Fällen 7 Tage. Die Ergebnisse dieser Teste mit repräsentativen Vertretern der erfindungsgemäßen Penicilline und Cephalosporine sind in der Tabelle 2 dargestellt.

Tabelle 2

In vivo Aktivität bei Mäusen

E. coli-Infektion (s.c. Applikation):

| Verbindung | $ED_{50}$ (mg/kg) |
|---|---|
| A | 5,5 |
| B | 4,5 |
| C | 0,6 |
| D | 3,0 |
| Azlocillin | >100 |
| Cefuroxim | >100 |

Es ist eine weitere Aufgabe der vorliegenden Erfindung, pharmazeutische Mittel zu schaffen, die bei der Behandlung infektiöser Krankheiten sowohl beim Menschen als auch beim Tier wertvoll sind.

Als bevorzugte pharmazeutische Zubereitungen seien Tabletten, Dragées, Kapseln, Granulate, Suppositorien, Lösungen, Suspensionen, Emulsionen, Salben, Gele, Cremes, Puder und Sprays genannt. Vorteilhafterweise wird der Wirkstoff in der Human- oder Tiermedizin oder ein Gemisch der verschiedenen Wirkstoffe der allgemeinen Formel I in einer Dosierung zwischen 5 und 500, vorzugsweise 10-200 mg/kg Körpergewicht je 24 Stunden verabreicht, gegebenenfalls in Form mehrerer Einzelgaben. Eine Einzelgabe enthält den oder die erfindungsgemäßen Wirkstoffe, vorzugsweise in Mengen von etwa 1 bis etwa 250, insbesondere 10 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive

Bakterien verhindert, gebessert und/oder geheilt werden und ebenso eine Förderung des Wachstums und eine Verbesserung der Verwertung des Futters erreicht werden.

Die folgenden Beispiele sollen die Erfindung näher erläutern:

Beispiel 1

D-α-[3-(2-p-Aminosulfonylanilino-4-hydroxy-5-pyrimidinyl)-ureido]-p-äthoxycarbonyloxy-benzylpenicillin-Natrium

2,8 g (0,01 Mol) 5-Amino-2-p-aminosulfonylanilino-4-hydroxy-pyrimidin werden in 100 ml trockenem Tetrahydrofuran aufgeschlämmt und mit 8 g Trimethylsilyldiäthylamin bis zur vollständigen Lösung zum Rückfluß erhitzt (10 bis 30 Minuten). Die Lösung wird im Vakuum zur Trockne eingeengt, wieder in 100 ml Tetrahydrofuran aufgenommen und unter Eiskühlung zu einer Lösung von 1,06 g Phosgen in 70 ml trockenem Tetrahydrofuran getropft. Man rührt 10 Minuten bei Raumtemperatur nach und engt dann im Vakuum zur Trockne ein. Das zurückbleibende Festprodukt wird unter Eiskühlung mit 160 ml Methanol versetzt, wobei Lösung eintritt. Nach kurzer Zeit fällt analysenreines 1-Hydro-5-(p-aminosulfonylanilino)-oxazolo[5,4-d]pyrimidin-2-on aus, das abgesaugt und getrocknet wird.

Man suspendiert 1,97 g (0,0045 Mol) D-α-Amino-p-äthoxy-carbonyloxy-benzylpenicillin in einem Lösemittelgemisch von 40 ml Tetrahydrofuran und 10 ml Wasser. Unter Eiskühlung fügt man bis zur Lösung Triäthylamin zu. Zu dieser Lösung wird portionsweise 1,4 g (0,0045 Mol) 1-Hydro-5-(p-aminosulfonylanilino)-oxazolo-[5,4-d]pyrimidin-2-on zugesetzt, wobei ein Absinken des pH-Wertes unter 7 gegebenenfalls durch Zugabe von Triäthylamin vermieden wird. Man rührt eine Stunde im Eisbad nach, läßt auf Raumtemperatur kommen, versetzt mit 20 ml Wasser und entfernt das Tetrahydrofuran im Vakuum. Die wäßrige Phase wird einmal mit Essigester bei pH 7,0 ausgeschüttelt und unter Eiskühlung mit 1 n Salzsäure auf pH 2,8 gestellt. Die ausgefallene D(-)-α-[3-(2-p-Aminosulfonylanilino-4-hydroxy-5-pyrimidinyl)ureido]-p-äthoxycarbonyloxy-benzylpenicillansäure wird abgesaugt und im Vakuum getrocknet. Man rührt in Dimethylformamid, setzt die berechnete Menge Natriumäthylhexanoat zu und fällt das Natriumsalz durch Verdünnen mit Äther aus.

Ausbeute: 2,12 g (61,5 % der Theorie),
IR-Spektrum: 1760, 1660, 1600, 1330, 1150 $cm^{-1}$;
NMR-Spektrum: (DMSO + $CD_3OD$), Signale bei ppm: 1,3 (t,3H), 1,55 (d,6H), 4,0 (s,1H), 4,25 (q, 2H) 5,40 (q,2H), 5,65 (s,1H), 7,15 (d,2H), 7,5 (d,2H), 7,7 (d,2H), 8,0 (d,2H), 8,35 (s,1H).

Beispiel 2

D-α-[3-(2-p-Aminosulfonylanilino-4-hydroxy-5-pyrimidinyl)-ureido]-p-acetoxy-benzylpenicillin-Natrium

a) 2,36 g (0,005 Mol) D(-)-α-[(2-p-Aminosulfonylanilino-4-hydroxy-5-pyrimidinyl)-ureido]-α-(p-hydroxy-phenyl)essigsäure werden in 50 ml Eisessig suspendiert und dazu 1,75 g (0,02 Mol) Acetylchlorid gegeben.
Die Reaktionsmischung wird 12 Stunden bei 20°C gerührt. Man gibt nochmals 1,75 g Acetylchlorid zu, erwärmt kurz auf 60°C und rührt danach 1 weitere Stunde bei 20°C.
Nach Zugabe von 100 ml Äther wird abgenutscht, das kristalline Produkt 2 mal mit jeweils 100 ml Äther ausgerührt, abgenutscht und getrocknet.
Man erhält 2,4 g (93 %) D(-)-α-[(2-p-Aminosulfonylanilino-4-hydroxy-5-pyrimidinyl)-ureido]-α-(p-acetoxy-phenyl)essigsäure.
Schmelzpunkt: 214°C (Zers.).

b) Zu einer Lösung von 2,2 g (0,00425 Mol) D(-)-α-[(2-p-Aminosulfonylanilino-4-hydroxy-5-pyrimidinyl)-ureido]-α-(p-acetoxy-phenyl)essigsäure in einem Gemisch von 50 ml Dimethylformamid und 10 ml Methylenchlorid werden 0,46 ml (0,00425 Mol) N-Methylmorpholin gegeben. Man kühlt auf -35°C ab, und tropft eine Lösung von 0,41 ml (0,00425 Mol) Chlorameisensäureäthylester in 2 ml Methylenchlorid zu.

Nach einer Reaktionszeit von 10 Minuten bei $-35^{o}C$ werden 1,34 g (0,00425 Mol) 6-Amino-penicillansäure-triäthylammoniumsalz, gelöst in 60 ml Methylenchlorid, zugegeben.

Man rührt 30 Minuten bei $-35^{o}C$, entfernt dann die Kühlung und läßt die Temperatur der Reaktionsmischung innerhalb von 30 Minuten auf $20^{o}C$ ansteigen.
Anschließend wird in ein Gemisch von 200 ml Wasser und 200 ml Methylacetat gegossen, der pH-Wert mit 1n Natronlauge auf 7 gestellt und die organische Phase abgetrennt.
Die wäßrige Phase wird unter Eiskühlung mit 1n Salzsäure auf pH 2,8 gestellt, die ausgefallene D(-)-α-[3-(2-p-Amino-sulfonylanilino-4-hydroxy-5-pyrimidinyl)ureido]-p-acetoxy-benzylpenicillansäure abgesaugt, mit Wasser gewaschen und getrocknet.
Man löst in Dimethylformamid, setzt die berechnete Menge Natrium-äthylhexanoat zu und fällt das Natriumsalz durch Zugabe von Äther aus.
Ausbeute: 2,1 g (67 % der Theorie),
IR-Spektrum: 1770, 1660, 1600, 1330, 1150 $cm^{-1}$,
NMR-Spektrum: (DMSO + $CD_3OD$), Signale bei ppm:
1,55 (d,6H), 2,20 (s,3H), 4,0 (s, 1H),
5,40 (q, 2H), 5,60 (s,1H), 7,05 (d, 2H),
7,45 (d,2H), 7,6 (d,2H), 7,95 (d,2H),
8,30 (s,1H)

## Beispiel 3

D-α-[3-(4-Hydroxy-2- {4'-hydroxycyclohexylamino} -5-pyrimidinyl)-ureido]-p-äthoxycarbonyloxy-benzylpenicillin-Natrium

1,12 g 5-Amino-4-hydroxy-2-(4'-hydroxycyclohexylamino)-pyrimidin (5 mMol) werden analog Beispiel 1 silyliert und mit Phosgen umgesetzt. Die Lösung des erhaltenen Produkts in Tetrahydrofuran wird zu einer wie in Beispiel 1 bereiteten Lösung

des dort verwendeten Aminopenicillin-derivates getropft. Die Aufarbeitung erfolgt analog Beispiel 1.

Ausbeute: 49 %;

IR-Spektrum: 1770, 1660, 1605, 1330, 1150 $cm^{-1}$.

NMR-Spektrum ($DMSO+CD_3OD$) Signale bei ppm:
1,3 (t,3H), 1,55 (d,6H), 1,75 (m,8H), 3,6-4,1 (m,1H + s,1H + m,1H), 4,15 (q,2H), 5,45 (q,2H), 7,15 (d,2H), 7,5 (d,2H), 8,0 (s,1H)

Analog den Beispielen 1 und 2 wurden die Penicilline der folgenden Tabelle synthetisiert:

| Beispiel | $R_1$ | $R_2$ | R | Ausbeute % | NMR-Spektren ($DMSO+CD_3OD$) Signale bei ppm |
|---|---|---|---|---|---|
| 4 | $CH_3O\underset{\underset{O}{\|}}{C}-$ | H | 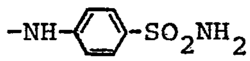 | 64,5 | 1,55 (d,6H), 3,90 3H), 4,05 (s,1H), 5,45 (q,2H), 5,60 (s,1H), 7,10 (d,2H) 7,45 (d,2H), 7,70 (d,2H), 7,95 (d,2H), 8,35 (s,1H) |
| 5 | $CH_3O\underset{\underset{O}{\|}}{C}-$ | H | 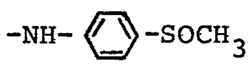 | 46 | 1,55 (d,6H), 2.7 (s,3H), 3,95 (s,3H), 4,0 (s,1H), 5,4 (q, 2H), 5,6 (s,1H), 7,15 (d,2H), 7,5 (d,2H), 7,75 (d,2H), 8,0 (d,2H), 8,35 (s, 1H) |
| 6 | $C_2H_5O\underset{\underset{O}{\|}}{C}-$ | H | 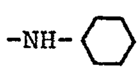 | 51 | 1,3 (t,3H), 1,4-2,0 (m,10+6H), 4,0 (m, 1H+ s,1H), 4,15 (q, 2H), 5,4 (q,2H), 5,65 (s,1H), 7,15 (d,2H), 7,5 (d,2H), 8,05 (s,1H) |

| Beispiel | $R_1$ | $R_2$ | R | Ausbeute % | NMR-Spektren ($DMSO+CD_3OD$) Signale bei ppm |
|---|---|---|---|---|---|
| 7 | $C_2H_5O\underset{\underset{O}{\|}}{C}-$ | H | $-NH(CH_2)_3OH$ | 54,5 | 1,3 (t,3H), 1,90 (m, 2H), 3,3 (m,2H), 3,65 (m,2H), 4,0 (s, 1H, 4,1 (q,2H), 5,45 (q,2H), 5,6 (s,1H), 7,15 (d,2H), 7,5 (d, 2H), 8,05 (s,1H) |
| 8 | $H_2N\underset{\underset{O}{\|}}{C}-$ | H | 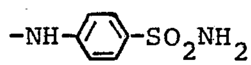 | 68 | 1,55 (d,6H), 4,05 (s,1H), 5,45 (q,2H + s,1H), 7,7 (d,2H), 7,95 (d,2H), 8,36 (s,1H) |

Beispiel 9

Natrium-7-{D-α-[(2-aminosulfonylanilino-4-hydroxy-5-pyrimidinyl)-ureido]-p-äthoxycarbonyloxy-phenylacetamido}-3-[(1-methyl-tetrazol-5-yl)-thiomethyl]-ceph-3-em-4-carboxylat

a) 2,8 g (0,01 Mol) 5-Amino-2-p-aminosulfonylanilino-4-hydroxy-pyrimidin werden in 100 ml trockenem Tetrahydrofuran aufgeschlämmt und mit 8 g Trimethylsilyldiäthylamin bis zur vollständigen Lösung zum Rückfluß erhitzt (10 bis 30 Minuten). Die Lösung wird im Vakuum zur Trockne eingeengt, wieder in 100 ml Tetrahydrofuran aufgenommen und unter Eiskühlung zu einer Lösung von 1,06 g Phosgen in 70 ml trockenem Tetrahydrofuran zugetropft. Man rührt 10 Minuten bei Raumtemperatur nach und engt dann im Vakuum zur Trockne ein. Das zurückbleibende Festprodukt wird unter Eiskühlung mit 160 ml Methanol versetzt, wobei Lösung eintritt. Nach kurzer Zeit fällt

analysenreines 1-Hydro-5-(p-aminosulfonylanilino)-oxazolo-/5,4-d/pyrimidin-2-on aus, das abgesaugt und getrocknet wird.

b) Man suspendiert 1,4 g (0,005 Mol) D(-)-α-Amino-(p-äthoxycarbonyloxy-phenyl)essigsäurehydrochlorid in 60 ml Tetrahydrofuran und fügt unter Eiskühlung 20 ml 0,5 n Natronlauge zu.
Die entstandene Lösung wird bei Raumtemperatur portionsweise mit 1,5 g (0,005 Mol) 1-Hydro-5-(p-aminosulfonylanilino)-oxazolo/5,4-d/pyrimidin-2-on versetzt, wobei ein pH-Wert von 8,0 - 8,3 eingehalten wird. Nach beendeter Zugabe wird nach einer Stunde bei Raumtemperatur nachgerührt.
Man versetzt die Reaktionsmischung mit 50 ml Wasser, stellt den pH-Wert mit 1n Salzsäure auf 3,0 und extrahiert 2 mal mit je 100 ml Essigsäureäthylester.
Die vereinigten organischen Phasen werden mit Wasser gewaschen, über Natriumsulfat getrocknet, im Vakuum eingedampft und der feste Rückstand mit Äther verrieben. Man erhält 2,4 g (88 % der Theorie) D(-)-α-/(2-p-Aminosulfonylanilino-4-hydroxy-5-pyrimidinyl)-ureido/-α-(p-äthoxycarbonyloxy-phenyl)-essigsäure.
Schmelzpunkt: 210°C (Zersetzung).

c) 2,7 g (0,005 Mol) D(-)-α-/(2-p-Aminosulfonylanilino-4-hydroxy-5-pyrimidinyl)-ureido/-α-(p-äthoxycarbonyloxy-phenyl)-essigsäure werden zusammen mit 2,47 g (0,005 Mol) 7-Amino-3-/(1-methyl-tetrazol-5-yl)-thiomethyl/-ceph-3-em-4-carbonsäurebenzhydrylester in einem Gemisch aus 70 ml trockenem Methylenchlorid und 30 ml Dimethylformamid gelöst. Man fügt unter Kühlung 1,1 g (0,005 Mol) Dicyclohexylcarbodiimid zu und rührt 6 Stunden unter Eiskühlung. Anschließend wird das Lösemittel im Vakuum entfernt. Der Rückstand wird zunächst mit 80 ml Methanol und dann zweimal mit jeweils 100 ml Methylenchlorid gut ausgerührt, wobei jeweils abgesaugt wird. Das zurückbleibende Festprodukt wird mit Äther gewaschen und getrocknet.

Der Benzhydrylester wird mit 10 ml Trifluoressigsäure und 3 ml Anisol auf übliche Weise gespalten und die Cephalosporansäure mit Natriumäthylhexanoat in Dimethylformamid in das Natriumsalz überführt.
Ausbeute an Natriumsalz: 2,57 g (59,5 %);
IR-Spektrum: 1760, 1665, 1600 $cm^{-1}$;
NMR-Spektrum (DMSO + $CD_3OD$) Signale bei ppm:
1,25 (t,3H), 3,40 (q,2H), 3,9 (s,3H), 4,0 (s,3H), 4,20 (q,2H), 4,80 (d,1H), 5,50 (s,1H), 5,6 (d,1H), 7,1 (d,2H), 7,4-7,7 (dd,4H), 7,9 (d,2H), 8,30 (s,1H).

Analog wurden die Cephalosporine der folgenden Tabelle synthetisiert:

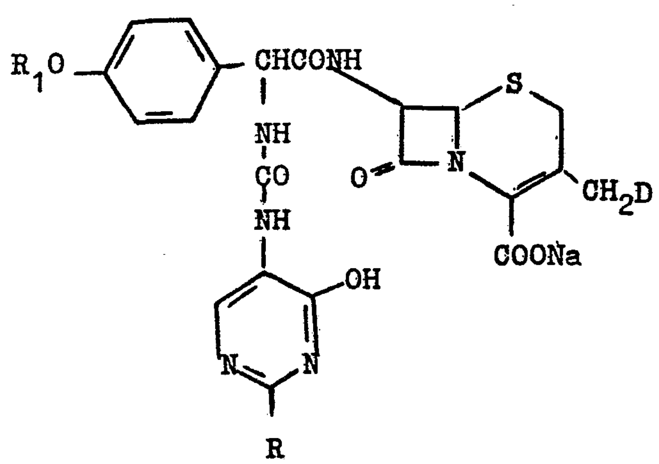

| Beispiel | $R_1$ | R | D | Ausbeute % | NMR-Spektrum ($DMSO+CD_3OD$) Signale bei ppm |
|---|---|---|---|---|---|
| 10 | 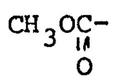 | 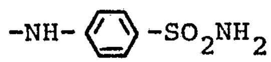 | $-OCOCH_3$ | 64 | 2,05 (s,3H), 3,45 (q,2H), 3,9o (s,3H), 4,80 (q,2H), 4,90 (d,1H), 5,55 (s,1H), 5,65 (d,1H), 7,0 (d,2H), 7,35-7,65 (dd,4H), 7,95 (d,2H), 8,35 (s,1H). |
| 11 | 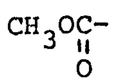 | 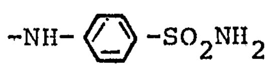 | 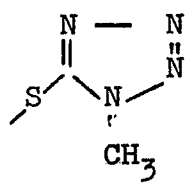 | 48,5 | 3,50 (q,2H), 3,95 (s,3H), 4,0 (s,3H), 4,3 (q,2H), 4,95 (d,1H) 5,50 (s,1H), 5,60 (d,1H), 7,1 (d,2H), 7,4-7,7 (dd,4H), 8,0 (d,2H), 8,38 (s,1H), |
| 12 | 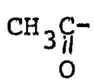 | 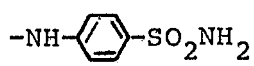 | 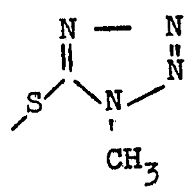 | 44 | 2,15 (s,3H), 3,50 (m,2H), 3,85 (s,3H), 4,25 (m,2H), 4,85 (d,1H), 5,5 (s,1H), 5,65 (d,1H), 7,0 (d,2H), 7,3-7,85 (m,6H), 8,35 (s,1H), |

| Beispiel | $R_1$ | R | D | Ausbeute % | NMR-Spektrum (DMSO+$CD_3OD$) Signale bei ppm |
|---|---|---|---|---|---|
| 13 | $C_2H_5O\underset{O}{\underset{\|}{C}}-$ | $-NH(CH_2)_3OH$ | 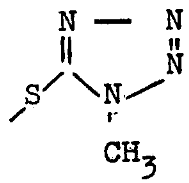 | 51 | 1,25 (t,3H), 1,90 (m,2H), 3,2 (m,2H), 3,45 (m,2H), 3,65 (m,2H), 3,95 (s,3H), 4,2 (m,2H + m,2H), 4,90 (d,1H), 5,5 (s,1H), 5,65 (d,1H), 7,1 (d,2H), 7,4 (d,2H), 8,35 (s,1H), |
| 14 | $C_2H_5O\underset{O}{\underset{\|}{C}}-$ | $-NHCH(CH_3)_2$ | 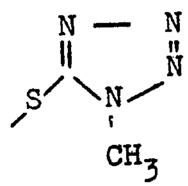 | 55 | 1,15 (d,6H), 1,3 (t,3H), 3,50 (q,2H), 3,8 (breites m, 1H), 3,95 (s,3H), 4,25 (m,4H), 4,90 (d,1H), 5,50 (s,1H), 5,65 (d,1H), 7,0-7,4 (m,4H), 8,05 (s,1H), |
| 15 | $H_2N\underset{O}{\underset{\|}{C}}-$ | 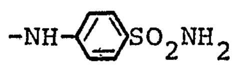 | 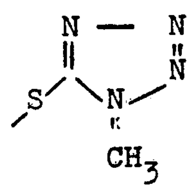 | 44 | 3,45 (q,2H), 3,95 (s,3H), 4,25 (m,2H), 4,95 (d,1H), 5,50 (s,1H), 5,65 (d,1H), 7,1-7,4 (m,4H), 7,65 (d,2H), 7,95 (d,2H), 8,36 (s,1H). |

Die Verbindungen der allgemeinen Formel I und I' lassen sich in die üblichen pharmazeutischen Anwendungsformen, wie Tabletten, Dragées, Kapseln oder Ampullen einarbeiten. Die Einzeldosis beträgt bei Erwachsenen im allgemeinen zwischen 50 und 1000 mg, vorzugsweise 100 bis 500 mg, die Tagesdosis zwischen 100 und 4000 mg, vorzugsweise 250 bis 2000 mg.

Beispiel I

Tabletten enthaltend D-α-[3-(2-p-Aminosulfonylanilino-4-hydroxy-5-pyrimidinyl)-ureido]-p-methoxycarbonyloxy-benzylpenicillin-Natrium

Ein Gemisch bestehend aus 2 kg Wirksubstanz, 5 kg Lactose, 1,8 kg Kartoffelstärke, 0,1 kg Magnesiumstearat und 0,1 kg Talk wird in üblicher Weise zu Tabletten gepreßt, derart, daß jede Tablette 200 mg Wirkstoff enthält.

Beispiel II

Dragées enthaltend D-α-[3-(2-p-Aminosulfonylanilino-4-hydroxy-5-pyrimidinyl)-ureido]-p-methoxycarbonyloxy-benzylpenicillin-Natrium

Analog Beispiel I werden Tabletten gepreßt, die anschließend in üblicher Weise mit einem Überzug bestehend aus Zucker, Kartoffelstärke, Talk und Tragant überzogen werden.

## Beispiel III

**Kapseln enthaltend D-α-[3-(2-p-Aminosulfonylanilino-4-hydroxy-5-pyrimidinyl)-ureido]-p-methoxycarbonyloxy-benzylpenicillin-Natrium**

5 kg Wirksubstanz werden in üblicher Weise in Hartgelatinekapseln gefüllt, derart, daß jede Kapsel 500 mg des Wirkstoffes enthält.

## Beispiel IV

**Trockenampullen enthaltend Natrium 7-{D-α-[3-(2-p-aminosulfonylanilino-4-hydroxy-5-pyrimidinyl)-ureido]-p-methoxycarbonyloxy-phenylacetamido}-3-[(1-methyl-tetrazol-5-yl)-thiomethyl]-ceph-3-em-4-carboxylat**

In einem aspetischen Bereich wurde 251 g Wirkstoff in 200 ml destilliertem Wasser zur Injektion aufgelöst. Die Lösung wurde durch ein Millipore-Filter (Porengröße 0,22 um, Produkt der Millipore Corporation, Bedford, USA) filtriert. Die Lösung wurde jeweils in einer Menge von 2,0 ml in 1000 Gläschen (Kapazität 10 ml) eingegossen und es wurde lyophilisiert. Die Gläschen wurden sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml in Ampullen abgefüllt und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare Zubereitung für die intravenöse Verabreichung erhalten wurde.

Destilliertes Wasser zur Injektion wurde in einer Menge von 20 ml in die Gläschen (Nr. A) gegossen und die Lösung wurde in einer 5%igen Lösung von Glucose für Injektionen (250 ml) aufgelöst. Auf diese Weise wurden Lösungen für die kontinuierliche Infusion hergestellt.

Analog sind Tabletten, Dragées, Kapseln und Ampullen erhältlich, die einen oder mehrere der übrigen Wirkstoffe der Formel I oder die physiologisch unbedenklichen Salze dieser Verbindungen enthalten.

## P a t e n t a n s p r ü c h e

1. Neue ß-Lactame der allgemeinen Formeln

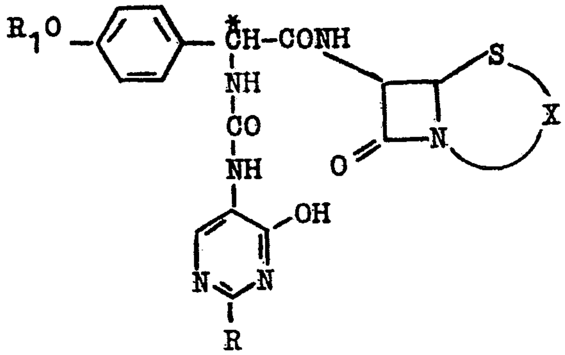

,(I)

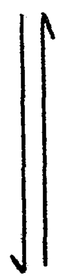

bzw.

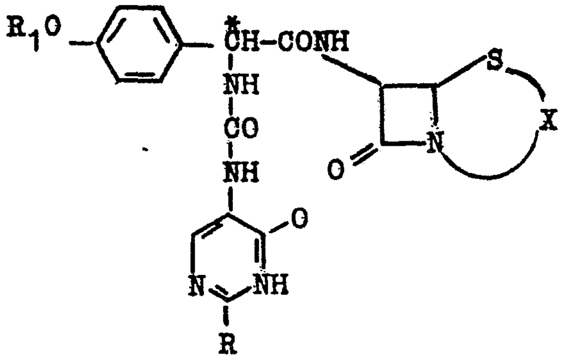

,(I')

in welchen
R, $R_1$ und X die folgenden Bedeutungen besitzen:

$R_1$ eine aliphatische Acyl- oder Alkoxycarbonylgruppe mit 2 bis 5 Kohlenstoffatomen oder die Aminocarbonylgruppe,

X die Gruppen

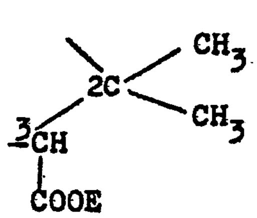

und

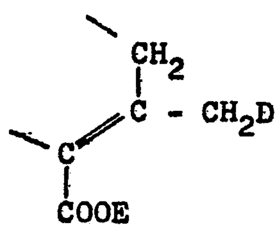

,

worin
D ein Wasserstoffatom, eine Acetoxy-, Aminocarbonyloxy-, Pyridinium oder 4-Aminocarbonyl-pyridiniumgruppe oder die Gruppe -SHet bedeutet, wobei Het die 1-Methyl-tetrazol-5-yl-, 1,2,4-Thiadiazol-5-yl-, 3-Methyl-1,2,4-thiadiazol-5-yl-, 1,3,4-Thiadiazol-2-yl-, 2-Methyl-1,3,4-thiadiazol-5-yl- oder die 4-Methyl-5,6-dioxo-1,2,4-triazin-3-yl-gruppe darstellt und

E ein Wasserstoffatom oder eine in vitro oder in vivo leicht spaltbare Schutzgruppe;

R ein Wasserstoffatom, die Cyclopropylgruppe, die 2'-Hydroxyäthylamino-, 3'-Hydroxypropylamino- oder 4'-Hydroxycyclohexylaminogruppe oder eine Gruppe der allgemeinen Formel

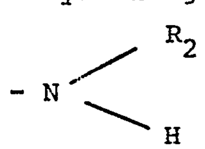

,worin $R_2$ Wasserstoff, eine verzweigte oder unverzweigte Alkyl- oder Alkenylgruppe mit 1 bis 4 Kohlenstoffatomen oder einen Cycloalkylrest mit 3 bis 6 Kohlenstoffatomen bedeutet,

R bedeutet weiter eine Gruppe der allgemeinen Formel

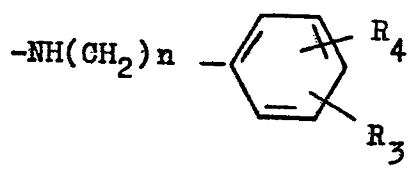

worin
n = o oder 1 und $R_3$ und $R_4$, die gleich oder verschieden sein können, Wasserstoffatome, eine Hydroxy-, Acetylamino-, Aminocarbonylamino-, Nitro-, Aminocarbonyl-, Cyan-, Methylsulfinyl-, Methylsulfonyl-, Aminosulfonyl- oder Methylaminosulfonylgruppe bedeuten, und, falls E ein Wasserstoffatom bedeutet, deren physiologisch verträgliche Salze mit anorganischen oder organischen Basen.

2. Neue ß-Lactame der allgemeinen Formeln

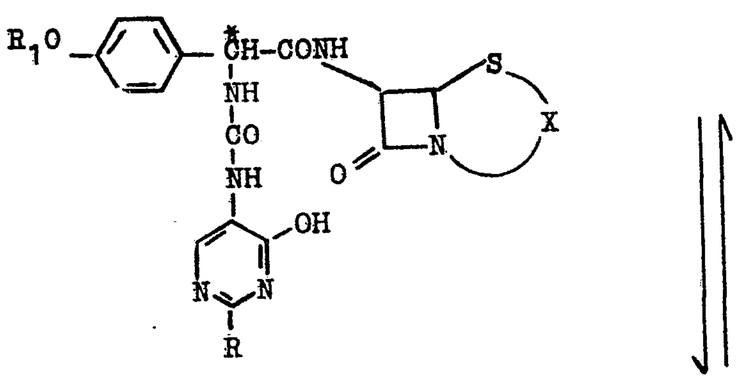

,(I)

bzw.

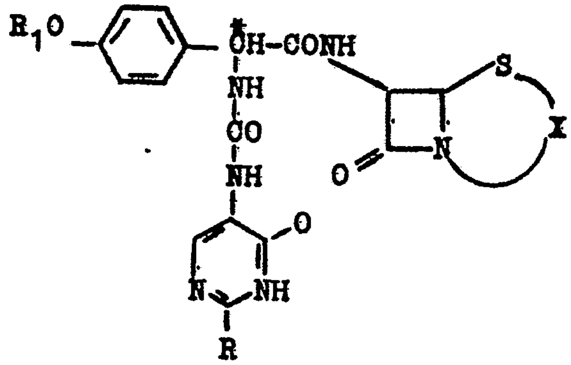

,(I')

in welchen

$R_1$ eine Methoxycarbonyl-, Äthoxycarbonyl-, Acetyl- oder Aminocarbonylgruppe bedeutet,

X wie im Anspruch 1 definiert ist, und in X das Symbol

E ein Wasserstoffatom, die Pivaloyloxymethylgruppe und

D ein Wasserstoffatom, die Acetoxygruppe oder die Gruppe -SHet darstellen, wobei Het die 1-Methyltetrazol-5-yl-, die 1,3,4-Thiadiazol-5-yl- oder die 2-Methyl-1,3,4-thiadiazol-5-yl-Gruppe bedeutet, und

R die in Anspruch 1 genannten Bedeutungen besitzt und, falls E ein Wasserstoffatom bedeutet, deren physiologisch verträgliche Salze mit anorganischen oder organischen Basen.

3. Neue ß-Lactame der allgemeinen Formeln I oder I' gemäß Anspruch 1 oder 2, dadurch gekennzeichnet, daß $R_1$ und X wie im Anspruch 2 definiert sind und
R entweder die Gruppe der allgemeinen Formel

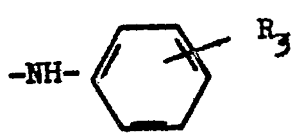

bedeutet, in der $R_3$ die Hydroxy-, Methylsulfinyl-, Methylsulfonyl-, Aminocarbonyl-, Aminocarbonylamino-, Aminosulfonyl- oder Methylaminosulfonylgruppe darstellt, oder R die m-Hydroxy-p-aminosulfonylanilino-, Cyclopropyl-, Propylamino-, Isopropylamino-, Cyclopentylamino-, Cyclohexylamino-, 3'-Hydroxypropylamino- oder 4'-Hydroxycyclohexylaminogruppe bedeutet und, falls E ein Wasserstoffatom bedeutet, deren physiologisch verträgliche Salze mit anorganischen oder organischen Basen.

4. Neue ß-Lactame der allgemeinen Formeln I bzw. I' gemäß den Ansprüchen 1 bis 3, dadurch gekennzeichnet, daß R, $R_1$ und X mit D die in den Ansprüchen 1, 2 oder 3 angegebenen Bedeutungen besitzen und E als leicht abspaltbare Gruppe die Benzyl-, Diphenylmethyl-, Trityl-, t-Butyl-, die 2,2,2-Trichloräthyl-, die Trimethylsilylgruppe oder eine Alkanoyloxyalkylgruppe mit 1 bis 5 Kohlenstoffatomen im Alkanoylrest, mit 1 bis 3 Kohlenstoffatomen im Alkylenrest oder die Phthalidyl- oder Indanylgruppe darstellt.

5. Neue ß-Lactame der allgemeinen Formel

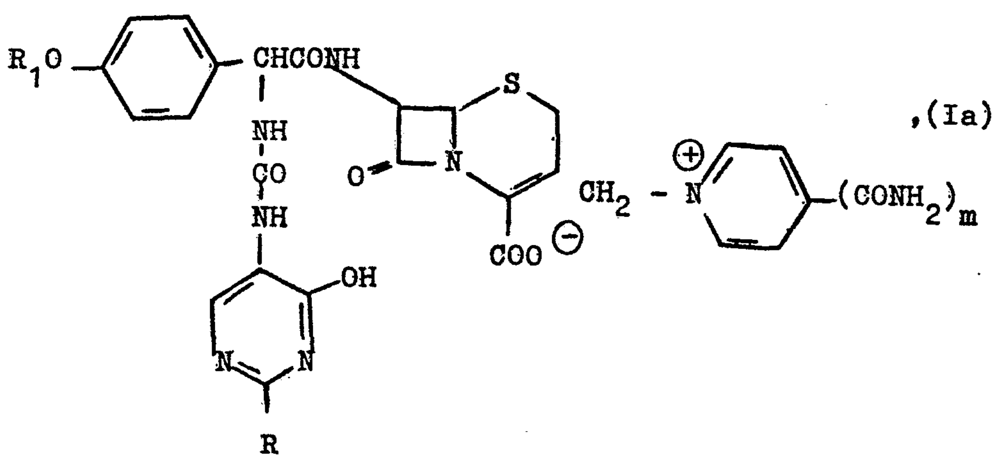

,(Ia)

in der
R und $R_1$ wie in den Ansprüchen 1 bis 4 definiert sind und m die Zahl 0 oder 1 bedeutet.

6. Als neue Verbindungen

a) D-α-[3-(2-p-Aminosulfonylanilino-4-hydroxy-5-pyrimidinyl)-ureido]-p-äthoxycarbonyloxy-benzylpenicillin-Natrium

b) D-α-[3-(2-p-Aminosulfonylanilino-4-hydroxy-5-pyrimidinyl)-ureido]-p-methylcarbonyloxy-benzylpenicillin-Natrium

c) Natrium-7-{D-α-[3-(2-p-aminosulfonylanilino-4-hydroxy-5-pyrimidinyl)-ureido]-p-methylcarbonyloxy-phenylacetamido}-3-[(1-methyl-tetrazol-5-yl)-thiomethyl]-ceph-3-em-4-carboxylat

d) Natrium-7-{D-α-[3-(2-p-aminosulfonylanilino-4-hydroxy-5-pyrimidinyl)-ureido]-p-äthoxycarbonyloxy-phenylacetamido}-3-[(1-methyl-tetrazol-5-yl)-thiomethyl]-ceph-3-em-4-carboxylat

und deren physiologisch verträgliche Salze mit anorganischen oder organischen Basen.

7. Arzneimittel gekennzeichnet durch einen Gehalt an einem oder mehreren Wirkstoffen der allgemeinen Formeln I oder I' bzw. Ia gemäß den Ansprüchen 1 bis 6 neben den üblichen Träger- und/oder Hilfsstoffen.

8. Verfahren zur Herstellung von neuen ß-Lactamen der allgemeinen Formeln

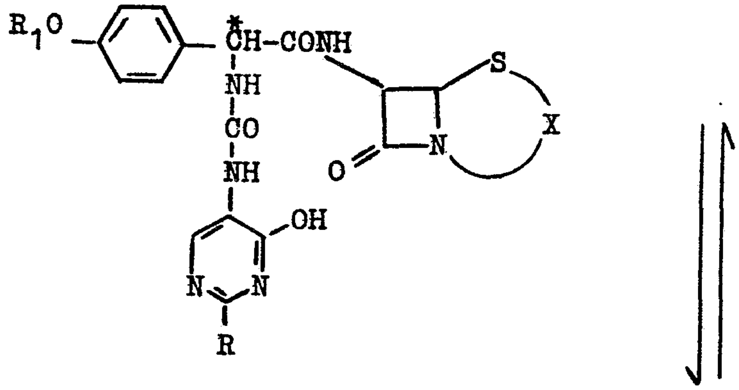

,(I)

bzw.

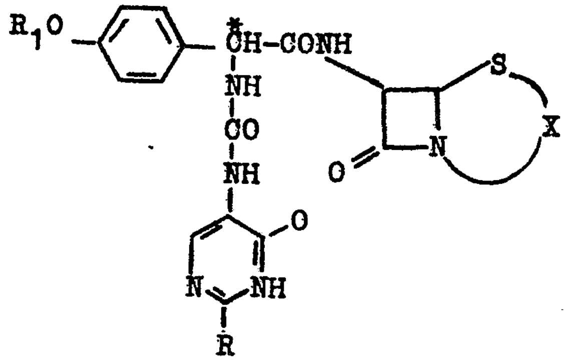

,(I')

in welchen
R, $R_1$ und X die folgenden Bedeutungen besitzen:

$R_1$ eine aliphatische Acyl- oder Alkoxycarbonylgruppe mit 2 bis 5 Kohlenstoffatomen oder die Aminocarbonylgruppe

X die Gruppen

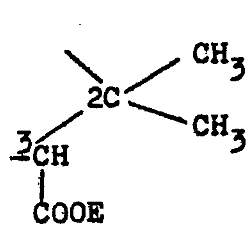

und

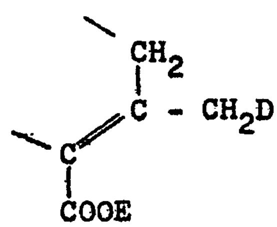

,

worin

D ein Wasserstoffatom, eine Acetoxy-, Aminocarbonyloxy-, Pyridinium oder 4-Aminocarbonyl-pyridiniumgruppe oder die Gruppe -SHet bedeutet, wobei Het die 1-Methyl-tetrazol-5-yl-, 1,2,4-Thiadiazol-5-yl-, 3-Methyl-1,2,4-thiadiazol-5-yl-, 1,3,4-Thiadiazol-2-yl-, 2-Methyl-1,3,4-thiadiazol-5-yl- oder die 4-Methyl-5,6-dioxo-1,2,4-triazin-3-yl-gruppe darstellt und

E ein Wasserstoffatom oder eine in vitro oder in vivo leicht spaltbare Schutzgruppe;

R ein Wasserstoffatom, die 3'-Hydroxypropylamino-, die Cyclopropylgruppe, die 2'-Hydroxyäthylamino-, 4'-Hydroxycyclohexylaminogruppe oder eine Gruppe der allgemeinen Formel

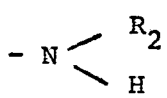 , worin $R_2$ Wasserstoff, eine verzweigte oder unverzweigte Alkyl- oder Alkenylgruppe mit 1 bis 4 Kohlenstoffatomen oder einen Cycloalkylrest mit 1 bis 6 Kohlenstoffatomen bedeutet,

R bedeutet weiter eine Gruppe der allgemeinen Formel

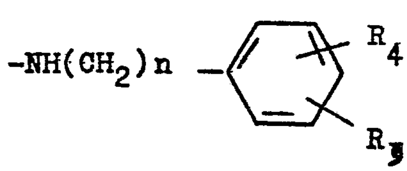

worin

n = o oder 1 und $R_3$ und $R_4$, die gleich oder verschieden sein können, Wasserstoffatome, eine Hydroxy-, Acetylamino-, Aminocarbonylamino-, Nitro-, Aminocarbonyl-, Cyan-, Methylsulfinyl-, Methylsulfonyl-, Aminosulfonyl- oder die Methylaminosulfonylgruppe bedeutet, und, falls E ein Wasserstoffatom darstellt, von deren physiologisch verträglichen Salzen mit anorganischen oder organischen Basen, dadurch gekennzeichnet, daß

a) zur Herstellung einer Verbindung der allgemeinen Formel I, bzw. I' in der D die angegebenen Bedeutungen mit Ausnahme der einer Pyridinium- oder Aminocarbonylpyridiniumgruppe besitzt, eine Verbindung der allgemeinen Formel

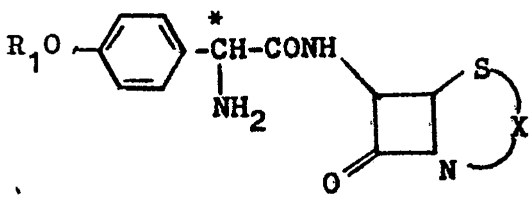 ,(II)

in der

$R_1$ und X die oben angegebenen Bedeutungen und D die oben angegebenen Bedeutungen mit Ausnahme der einer Pyridinium- oder Aminocarbonylpyridiniumgruppe besitzt, mit einem Pyrimidinderivat der allgemeinen Formel

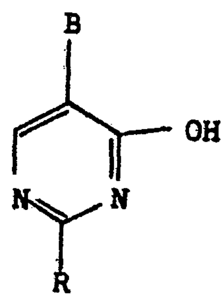 , (III)

in der

R wie oben definiert ist und B die Gruppen -NCO, -NHCOCl, -NHCOBr oder -NH-COO-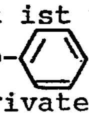-$NO_2$, bedeutet, oder mit Gemischen solcher Pyrimidinderivaten der allgemeinen Formel III, in der B teils die eine und teils die andere der vorstehend genannten Bedeutungen besitzt, in einem Lösungsmittel und bei einem pH-Bereich zwischen 2,0 und 9,0 bei Temperaturen zwischen $-20^{\circ}C$ und $+50^{\circ}C$, umgesetzt wird, oder

b) zur Herstellung einer Verbindung der allgemeinen Formel I bzw. I', in der D die oben angegebenen Bedeutungen mit Ausnahme der einer Pyridinium- oder Aminocarbonylpyridiniumgruppe besitzt, eine Ureidocarbonsäure der allgemeinen Formel

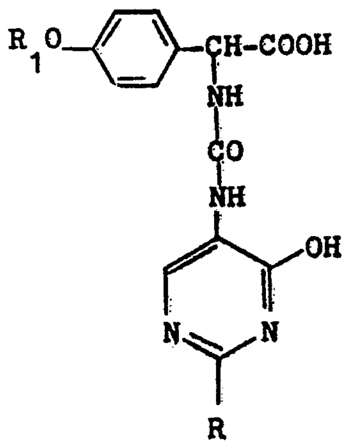

,(IV)

in der
R und $R_1$ die oben angegebenen Bedeutungen besitzen, oder ihre Salze oder reaktiven Derivate, mit Verbindungen der allgemeinen Formel

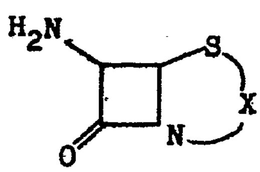

,(V)

in der
X die oben angegebenen Bedeutungen mit Ausnahme der Formel, in der D für Pyridinium oder Aminopyridinium steht, innehat, zwischen $-40^{\circ}C$ und $+40^{\circ}C$ in Gegenwart eines Lösungsmittels und gegebenenfalls in Gegenwart einer Base umgesetzt wird oder,

c) zur Herstellung von Cephalosporinderivaten der allgemeinen Formeln I bzw. I', in der D die Bedeutungen einer -S-Het-, Pyridinium- oder 4-Aminocarbonylpyridiniumgruppe besitzt, eine Verbindung der allgemeinen Formel

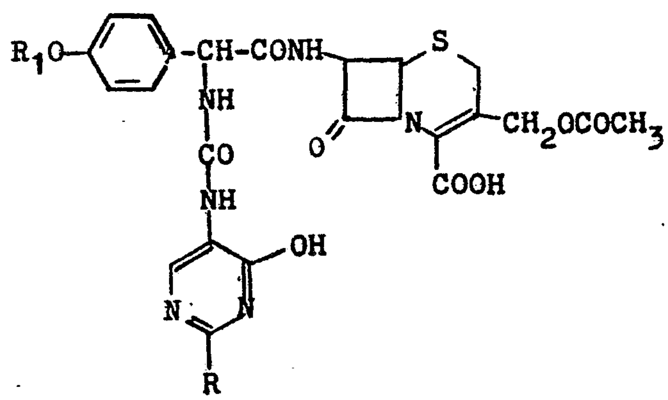 ,(VI)

die durch die allgemeinen Formeln I bzw. I' mitumfaßt wird und in der R und $R_1$ die oben angegebenen Bedeutungen haben, entweder mit einer Verbindung der allgemeinen Formel

$$Het - S - M \quad ,(VII)$$

in der Het die oben angegebenen Bedeutungen hat und M für ein Wasserstoffatom oder ein Alkalimetall oder ein Erdalkalimetall steht oder mit Pyridin bzw. 4-Aminocarbonylpyridin in einem organischen Lösungsmittel oder in Wasser oder in Gemischen dieser Lösungsmittel bei einem pH-Wert der Reaktionslösung zwischen 2 - 10, vorteilhafterweise zwischen 4-8, bei einer Temperatur im Bereich von 0° bis 100°C umgesetzt wird und gewünschtenfalls anschließend eine so hergestellte Verbindung der allgemeinen Formeln I oder I', in denen E für eine in vitro leicht abspaltbare Schutzgruppe steht, in die freie Carbonsäure der allgemeinen Formeln I bzw. I', in der

E ein Wasserstoffatom ist, überführt wird und/oder eine Verbindung der allgemeinen Formeln I oder I', in der E ein Wasserstoffatom bedeutet, in ihre Ester oder, mittels anorganischen oder organischen Basen, in die entsprechenden Salze übergeführt wird.

9. Verfahren gemäß Anspruch 8a, dadurch gekennzeichnet, daß eine Verbindung der allgemeinen Formel II, in der E Wasserstoff bedeutet, oder eines ihrer Salze mit anorganischen oder organischen Basen mit einer Verbindung der allgemeinen Formel III,

a) in Wasser oder in mit Wasser mischbaren Lösungsmitteln in Gegenwart von Wasser in einem pH-Bereich von 6,5 bis 8,0, oder
b) in wasserfreien Lösungsmitteln oder
c) in einem Gemenge aus Wasser und mit Wasser nicht mischbaren Lösungsmitteln in einem pH-Bereich zwischen 6,5- und 8,0

umgesetzt wird, oder, daß eine Verbindung der allgemeinen Formel II, in der E eine Silylgruppe oder eine andere leicht abspaltbare Schutzgruppe bedeutet, mit einer Verbindung der allgemeinen Formel III in einem wasser- und hydroxylgruppenfreien bzw. aprotischen Lösungsmittel, gegebenenfalls in Gegenwart einer Base, umgesetzt wird.

10. Verfahren gemäß Anspruch 8b, dadurch gekennzeichnet, daß als reaktive Derivate der Ureidocarbonsäuren der allgemeinen Formel IV deren Säureanhydride, deren reaktive Ester oder reaktive Amide oder deren Säurehalogenide, als reaktive Ester der Verbindungen der allgemeinen Formel V der Diphenylmethylester, der t-Butylester, der Trimethylsilylester oder das N,O-Bis-trimethylsilylderivat verwendet werden und die Umsetzung in Gegenwart einer Base und/oder eines organischen Lösungsmittels und/oder eines Kondensationsmittels erfolgt und, gewünschtenfalls, das so erhaltene Produkt der

allgemeinen Formel I, in der E eine abspaltbare Schutzgruppe bedeutet, anschließend in eine Verbindung der allgemeinen Formel I übergeführt wird, in der E ein Wasserstoffatom darstellt.

11. Verfahren gemäß Anspruch 8 zur Herstellung einer Verbindung der allgemeinen Formel I oder I', worin in X das Symbol E den Pivaloyloxymethylrest bedeutet, dadurch gekennzeichnet, daß eine Verbindung der allgemeinen Formel I oder I', in der E ein Wasserstoffatom darstellt, in ihr Alkalisalz übergeführt und dieses mit einem Pivaloyloxymethylhalogenid der allgemeinen Formel

$$Hal-CH_2-O-\underset{\underset{O}{\|}}{C}-C-(CH_3)_3,$$

worin Hal für Chlor, Brom oder Jod steht, in einem Lösungsmittel bei Temperaturen zwischen 20 und 45$^{o}$C umgesetzt wird.

Patentanprüche für das Benennungsland Österreich

1. Verfahren zur Herstellung von neuen ß-Lactamen der allgemeinen Formeln

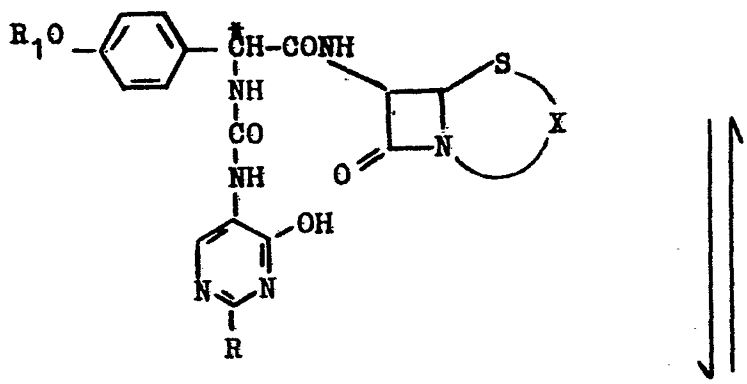

,(I)

bzw.

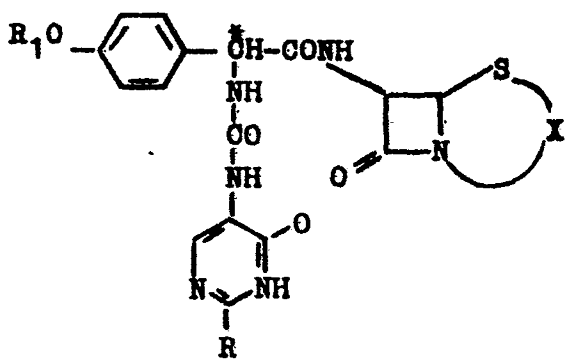

,(I')

in welchen

R, $R_1$ und X die folgenden Bedeutungen besitzen:

$R_1$ eine aliphatische Acyl- oder Alkoxycarbonylgruppe mit 2 bis 5 Kohlenstoffatomen oder die Aminocarbonylgruppe

X die Gruppen

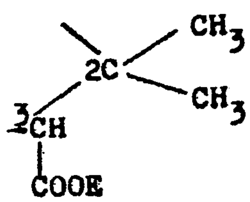

und

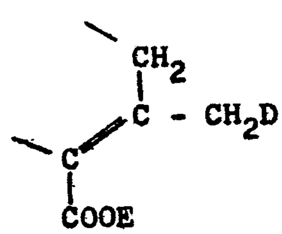

,

worin

D ein Wasserstoffatom, eine Acetoxy-, Aminocarbonyloxy-, Pyridinium oder 4-Aminocarbonyl-pyridiniumgruppe oder die Gruppe -SHet bedeutet, wobei Het die 1-Methyl-tetrazol-5-yl-, 1,2,4-Thiadiazol-5-yl-, 3-Methyl-1,2,4-thiadiazol-5-yl-, 1,3,4-Thiadiazol-2-yl-, 2-Methyl-1,3,4-thiadiazol-5-yl- oder die 4-Methyl-5,6-dioxo-1,2,4-triazin-3-yl-gruppe darstellt und

E ein Wasserstoffatom oder eine in vitro oder in vivo leicht spaltbare Schutzgruppe;

R ein Wasserstoffatom, die 3'-Hydroxypropylamino-, die Cyclopropylgruppe, die 2'-Hydroxyäthylamino-, 4'-Hydroxycyclohexylaminogruppe oder eine Gruppe der allgemeinen Formel

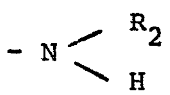 , worin $R_2$ Wasserstoff, eine verzweigte oder unverzweigte Alkyl- oder Alkenylgruppe mit 1 bis 4 Kohlenstoffatomen oder einen Cycloalkylrest mit 1 bis 6 Kohlenstoffatomen bedeutet,

R bedeutet weiter eine Gruppe der allgemeinen Formel

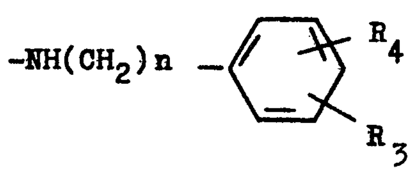

worin

n = o oder 1 und $R_3$ und $R_4$, die gleich oder verschieden sein können, Wasserstoffatome, eine Hydroxy-, Acetylamino-, Aminocarbonylamino-, Nitro-, Aminocarbonyl-, Cyan-, Methylsulfinyl-, Methylsulfonyl-, Aminosulfonyl- oder die Methylaminosulfonylgruppe bedeutet, und, falls E ein Wasserstoffatom darstellt, von deren physiologisch verträglichen Salzen mit anorganischen oder organischen Basen, dadurch gekennzeichnet, daß

a) zur Herstellung einer Verbindung der allgemeinen Formel I, bzw. I' in der D die angegebenen Bedeutungen mit Ausnahme der einer Pyridinium- oder Aminocarbonylpyridiniumgruppe besitzt, eine Verbindung der allgemeinen Formel

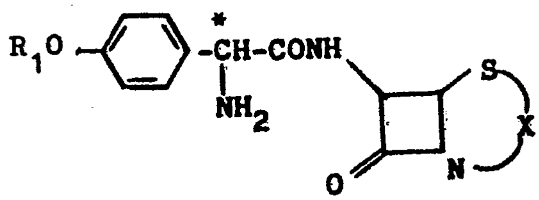

,(II)

in der
$R_1$ und X die oben angegebenen Bedeutungen und
D die oben angegebenen Bedeutungen mit Ausnahme der einer Pyridinium- oder Aminocarbonylpyridiniumgruppe besitzt, mit einem Pyrimidinderivat der allgemeinen Formel

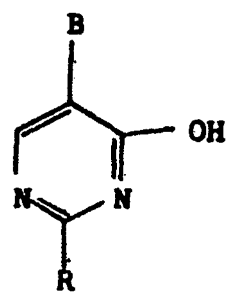

, (III)

in der
R wie oben definiert ist und B die Gruppen -NCO, -NHCOCl, -NHCOBr oder -NH-COO-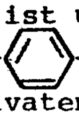-$NO_2$, bedeutet, oder mit Gemischen solcher Pyrimidinderivaten der allgemeinen Formel III, in der B teils die eine und teils die andere der vorstehend genannten Bedeutungen besitzt, in einem Lösungsmittel und bei einem pH-Bereich zwischen 2,0 und 9,0 bei Temperaturen zwischen -20°C und +50°C, umgesetzt wird, oder

b) zur Herstellung einer Verbindung der allgemeinen Formel I bzw. I', in der D die oben angegebenen Bedeutungen mit Ausnahme der einer Pyridinium- oder Aminocarbonylpyridiniumgruppe besitzt, eine Ureidocarbonsäure der allgemeinen Formel

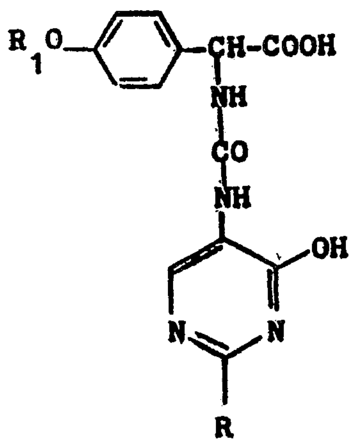

,(IV)

in der
R und $R_1$ die oben angegebenen Bedeutungen besitzen, oder ihre Salze oder reaktiven Derivate, mit Verbindungen der allgemeinen Formel

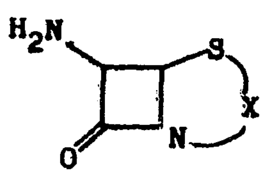

,(V)

in der
X die oben angegebenen Bedeutungen mit Ausnahme der Formel, in der D für Pyridinium oder Aminopyridinium steht, innehat, zwischen $-40^{o}C$ und $+40^{o}C$ in Gegenwart eines Lösungsmittels und gegebenenfalls in Gegenwart einer Base umgesetzt wird oder,

c) zur Herstellung von Cephalosporinderivaten der allgemeinen Formeln I bzw. I', in der D die Bedeutungen einer -S-Het-, Pyridinium- oder 4-Aminocarbonylpyridiniumgruppe besitzt, eine Verbindung der allgemeinen Formel

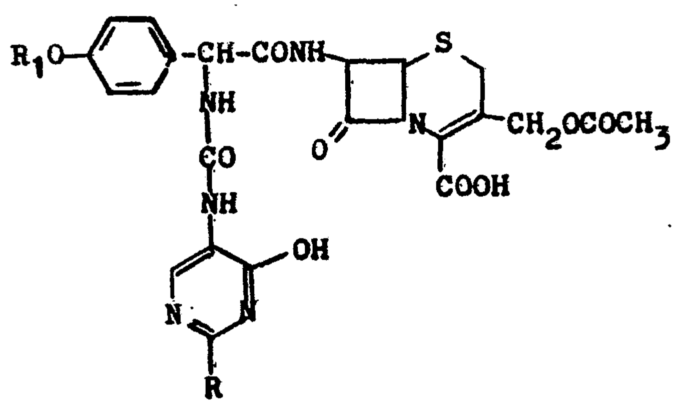

,(VI)

die durch die allgemeinen Formeln I bzw. I' mitumfaßt wird und in der R und $R_1$ die oben angegebenen Bedeutungen haben, entweder mit einer Verbindung der allgemeinen Formel

Het - S - M ,(VII)

in der Het die oben angegebenen Bedeutungen hat und M für ein Wasserstoffatom oder ein Alkalimetall oder ein Erdalkalimetall steht oder mit Pyridin bzw. 4-Aminocarbonylpyridin in einem organischen Lösungsmittel oder in Wasser oder in Gemischen dieser Lösungsmittel bei einem pH-Wert der Reaktionslösung zwischen 2 - 10, vorteilhafterweise zwischen 4-8, bei einer Temperatur im Bereich von $0^o$ bis $100^oC$ umgesetzt wird und gewünschtenfalls anschließend eine so hergestellte Verbindung der allgemeinen Formeln I oder I', in denen E für eine in vitro leicht abspaltbare Schutzgruppe steht, in die freie Carbonsäure der allgemeinen Formeln I bzw. I', in der

E ein Wasserstoffatom ist, überführt wird und/oder eine Verbindung der allgemeinen Formeln I oder I', in der E ein Wasserstoffatom bedeutet, in ihre Ester oder, mittels anorganischen oder organischen Basen, in die entsprechenden Salze übergeführt wird.

2. Verfahren gemäß Anspruch 1a, dadurch gekennzeichnet, daß eine Verbindung der allgemeinen Formel II, in der E Wasserstoff bedeutet, oder eines ihrer Salze mit anorganischen oder organischen Basen mit einer Verbindung der allgemeinen Formel III,

a) in Wasser oder in mit Wasser mischbaren Lösungsmitteln in Gegenwart von Wasser in einem pH-Bereich von 6,5 bis 8,0, oder
b) in wasserfreien Lösungsmitteln oder
c) in einem Gemenge aus Wasser und mit Wasser nicht mischbaren Lösungsmitteln in einem pH-Bereich zwischen 6,5- und 8,0

umgesetzt wird, oder, daß eine Verbindung der allgemeinen Formel II, in der E eine Silylgruppe oder eine andere leicht abspaltbare Schutzgruppe bedeutet, mit einer Verbindung der allgemeinen Formel III in einem wasser- und hydroxylgruppenfreien bzw. aprotischen Lösungsmittel, gegebenenfalls in Gegenwart einer Base, umgesetzt wird.

3. Verfahren gemäß Anspruch 1b, dadurch gekennzeichnet, daß als reaktive Derivate der Ureidocarbonsäuren der allgemeinen Formel IV deren Säureanhydride, deren reaktive Ester oder reaktive Amide oder deren Säurehalogenide, als reaktive Ester der Verbindungen der allgemeinen Formel V der Diphenylmethylester, der t-Butylester, der Trimethylsilylester oder das N,O-Bis-trimethylsilylderivat verwendet werden und die Umsetzung in Gegenwart einer Base und/oder eines organischen Lösungsmittels und/oder eines Kondensationsmittels erfolgt und, gewünschtenfalls, das so erhaltene Produkt der

allgemeinen Formel I, in der E eine abspaltbare Schutzgruppe bedeutet, anschließend in eine Verbindung der allgemeinen Formel I übergeführt wird, in der E ein Wasserstoffatom darstellt.

4. Verfahren gemäß Anspruch 1 zur Herstellung einer Verbindung der allgemeinen Formel I oder I', worin in X das Symbol E den Pivaloyloxymethylrest bedeutet, dadurch gekennzeichnet, daß eine Verbindung der allgemeinen Formel I oder I', in der E ein Wasserstoffatom darstellt, in ihr Alkalisalz übergeführt und dieses mit einem Pivaloyloxymethylhalogenid der allgemeinen Formel

$$Hal-CH_2-O-\underset{\underset{O}{\|}}{C}-C-(CH_3)_3,$$

worin Hal für Chlor, Brom oder Jod steht, in einem Lösungsmittel bei Temperaturen zwischen 20 und 45°C umgesetzt wird.

0044992

Europäisches Patentamt

# EUROPÄISCHER RECHERCHENBERICHT

Nummer der Anmeldung

EP 81 10 5441

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | EP - A - 0 006 532 (KARL THOMAE)<br>* Seiten 43-56; Ansprüche *<br>-- | 1-11 | C 07 D 501/20<br>499/78<br>A 61 K 31/545<br>31/43//<br>C 07 D 498/04<br>239/48 |
| | EP - A - 0 003 814 (KARL THOMAE)<br>* Seiten 1-21; Ansprüche *<br>-- | 1-11 | |
| P | EP - A - 0 022 494 (KARL THOMAE)<br>* Seiten 135-151; Ansprüche *<br>---- | 1-11 | |

RECHERCHIERTE SACHGEBIETE (Int. Cl.³)

C 07 D 501/20
501/36
501/22
501/24
501/32
499/78

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 06-11-1981 | LUYTEN |